# EUROPEAN PATENT SPECIFICATION

(11) **EP 3 025 257 B1**
(45) Date of publication and mention of the grant of the patent: **06.02.2019**
(21) Application number: 14829161.0
(22) Date of filing: 25.07.2014
(51) Int. Cl.: G06F 17/30, G06F 21/00, G01N 33/48, G06F 19/00, G06F 21/62, G16H 10/40, G16H 10/60, H04L 29/06

(54) **SYSTEMS AND METHODS FOR A DISTRIBUTED CLINICAL LABORATORY**
SYSTEME UND VERFAHREN FÜR EIN VERTEILTES KLINISCHES LABOR
SYSTÈMES ET PROCÉDÉS POUR UN LABORATOIRE CLINIQUE RÉPARTI

(30) Priority: 25.07.2013 US 201361858604 P
(43) Date of publication of application: 01.06.2016
(73) Proprietor: Theranos IP Company, LLC, Newark, CA 94560 (US)
(72) Inventor: BALWANI, Sunny, Palo Alto, California 94304 (US)
(74) Representative: Avidity IP
(86) International application number: PCT/US2014/048314
(87) International publication number: WO 2015/013688

(56) References cited:
- US-A1- 2005 027 995
- US-A1- 2011 166 890
- US-A1- 2011 178 814
- US-A1- 2011 295 517
- US-A1- 2012 232 367
- US-A1- 2013 156 286

## Description

### BACKGROUND

Information management in a clinical laboratory is generally centered around a laboratory information system (LIS). An LIS is typically a specialized database system that is traditionally configured to receive data from laboratory analytical instruments that are physically in the same clinical laboratory as the LIS. The LIS can provide functions such as automated reporting of test results, workflow management, and/or sample tracking. Known laboratory information systems are commercially available under the trade names Epix, Beacon, Sunquest, or Labdeck.

In a traditional LIS implementation, data usually travels from the laboratory analytical instruments to the laboratory's LIS in one of two ways. When a data requisition comes in, the system processes the requisition based typically on a barcode on the sample tube. When a barcoded sample tube is processed at a laboratory analyzer, the analyzer generates test results regarding analyte levels and/or other characteristics about the sample. The device then associates the test results with the barcode associated with the sample. Typically, the barcode is not necessarily patient ID, just an identifier for that sample vessel, and thus the analyzer typically does not know anything about the patient. The analyzer runs the sample with the barcode attached to it and then gives the results for the sample associated with that barcode.

Historically, up to 90% or more of laboratory test results managed by an LIS are faxed to the physician and thus the traditional laboratory test result handling and reporting is not highly automated or efficient. Additionally, most clinical laboratories are constrained by the traditional LIS used in such laboratories where the LIS is connected primarily by physical wired connections or other local communication protocols to analyzers that must also be physically in the same facility or in close proximity to the LIS of the clinical laboratory. Some such as rs232 are also limited in wire length. Due in part to this legacy infrastructure and traditional information handling paradigms, existing LIS implementations have various limitations which constrain their ability to handle and process data efficiently.

### SUMMARY

The disadvantages associated with the prior art are overcome by at least some of the embodiments described herein. The invention is defined by the independent claims. Further embodiments are defined by the dependent claims.

In at least one exemplary embodiment herein, a system is provided for expanding the sources from which data comes to LIS and un-tethering the LIS from working with only local analytical devices. At least one or more embodiments are provided wherein the analytical devices are not limited to those that connect to the LIS through a wired connection in the same physical facility, wirelessly in the same physically facility, or those devices that can be physically inspected by the laboratory director. In one embodiment, the sample processing device runs the sample, associates it with the barcode (or information conveyed by the barcode), and then generates the information/data. The information/data is transmitted to LIS which then puts it all together based on barcode information such as but not limited to sample ID. LIS may put it together based on the same requisition, the same visit, some other grouping, or other criteria. The typical output is a report that the lab director can review and recommend the appropriate action such as but not limited to out of range action, panic value action, etc... If values are in-range, then the information may be loaded into the electronic medical records (EMR) system. In at least one exemplary embodiment, the non-local devices that communicate with the LIS include at least one sample processing device, which is not an analyzer.

In at least one exemplary embodiment described herein, a method of transferring data between health information systems is provided. The method comprises receiving electronic sample processing data at a laboratory information system, wherein the data originates from at least one sample processing unit at a location remote from the laboratory information system and wherein the data traverses one or more wide area networks before reaching the laboratory information system. The method may include verifying sample processing data integrity; ensuring that the data is in a laboratory information system file format at a point in time after the data has been received by the laboratory information system; and storing data into the electronic medical records system by way of an interface between an electronic medical record system and the laboratory information system, wherein storing the data further comprises ensuring that the data is in an electronic medical record system file format.

It should be understood that embodiments in this disclosure may be adapted to have one or more of the features described below. In one non-limiting example, the sample processing units are physically distant from one another, and wherein a data pathway to the laboratory information system from at least one of the sample processing units comprises traversing: a) at least one wide area network, b) a central database collecting said sample processing data, and c) a listener application configured to process sample processing data from the central database for paired sample processing unit data. Optionally, verifying comprises using public key/private key encryption and decryption. Optionally, verifying comprises using at least one certificate for certificate authentication. Optionally, verifying comprises using at least one electronic certificate for certificate authentication. Optionally, certificate authentication comprises authenticating authorship of the certificate. Optionally, verifying extends oversight of testing integrity from an authorized laboratory to include oversight of the distributed, sample processing units. Optionally, the laboratory information system file format and the electronic medical record system file format are heterogeneous.

In at least one exemplary embodiment described herein, a method is provided for collecting data in a distributed laboratory system. The method comprises transmitting electronic sample processing data from at least one of a plurality of distributed, sample processing units to a laboratory information system having an interface for communicating over a computer network; wherein a data pathway for sample processing data from the one of the sample processing units to the laboratory information system comprises traversing at least one wide area network, wherein the data along the data pathway is handled by at least: a central database collecting said sample processing data from a plurality of sample processing units, and a listener application configured to processing data from the central database for paired sample processing unit data.

In at least one exemplary embodiment described herein, a method is provided for a distributed laboratory system, the method comprising: using an listener application for directing data from a non-local biological sample processing unit to a laboratory information system (LIS), wherein the client application is paired to gather data received from at least one or more non-local sample processing units; using certificate authentication to verify pairing of data from the sample processing unit and the listener application; wherein data pathway for sample processing data from the sample processing unit to the laboratory information system comprises at least one wide area network.

It should be understood that embodiments in this disclosure may be adapted to have one or more of the features described below. In one non-limiting example, the data pathway comprises an indirect connection between the source and the destination. Optionally, test results are entered into the laboratory information system before being available on an electronic medical records system. Optionally, test results are certified by a licensed professional before being available on an EMR system. Optionally, test results are entered into the EMR by way of first entering the results into the LIS which then integrates with the EMR. Optionally, the sample processing units are laboratory-waived devices wherein results are reviewed by LIS prior to being available in the EMR. Optionally, the method further comprises providing oversight from laboratory to the distributed, sample processing units. Optionally, the method further comprises checking when controls were run on one or more of the sample processing units. Optionally, the method further comprises pushing quality control out to the sample processing units to tell one or more of them to run calibrator(s) or to shut it down or take it offline until a calibrator is run or until a control cartridge is run. Optionally, the method further comprises pushing quality control out to the sample processing units to tell one or more of them to shut it down until someone runs a calibrator. Optionally, the method further comprises pushing quality control out to the sample processing units to tell one or more of them to shut it down until a control cartridge is run. Optionally, a physical wired connection is not required for the client application to send LIS the sample processing data from the sample processing unit. Optionally, connection to LIS is wireless without a data broker. Optionally, connection to LIS uses a cloud server to function as a data broker. Optionally, connection to LIS uses a pairing mechanism that associates certain sample processing units on non-local computer networks with certain listener applications. Optionally, an administrator sets which machines are in the testing environment. Optionally, an administrator searches the network to see which sample processing units are in a designated environment. Optionally, if sample processing units are not on same LAN, they are still accessible on WAN. Optionally, the listener is only listening for its designated set of machines. Optionally, a connection to LIS is to send data as a reference lab. Optionally, connection to LIS to send data through a gateway to the LIS. Optionally, connection to LIS to send data that has been reviewed to laboratory so that an authorized agent at the laboratory can certify the data. Optionally, a data connection is from analyzer device to LIS. Optionally, a data connection is from reference lab to another lab. Optionally, a data connection is from a laboratory providing service directly to a doctor.

In at least one exemplary embodiment described herein, a method is provided for a distributed laboratory system, the method comprising: using certificate authentication to verify pairing of a biological sample processing unit and a database network connected device at a destination laboratory; wherein data pathway for sample processing data from the sample processing units to the laboratory information system comprises at least one wide area network.

In at least one exemplary embodiment described herein, a method is provided for a distributed laboratory system, the method comprising: using certificate authentication to verify pairing of a biological sample processing unit and a database network connected device at a destination laboratory; wherein data pathway for sample processing data from the sample processing units to the laboratory information system comprises at least one wide area network; wherein regardless of where the sample processing unit is located, results are entered into the laboratory information system prior to being available in the electronic medical records system.

In at least one exemplary embodiment described herein, a laboratory system is provided comprising a plurality of distributed, sample processing units, each having at least one interface for communicating over at least one computer network; a laboratory information system configured to collect test results for samples processed by the distributed, sample processing units; a server comprising an interface for receiving sample processing data from at least one of the distributed, sample processing units; a client application on a programmable drive operably in communication with the server and operably in communication the laboratory information system, whereby the server provides sample processing data from only those sample processing units paired with the client application; wherein the client application allows for instructions to be sent to control the sample processing units and for monitoring operational status of one or more sample processing units whereby authorized oversight of the sample processing units is provided through the use of the client application to control and monitor the sample processing units.

It should be understood that embodiments in this disclosure may be adapted to have one or more of the features described below. In one non-limiting example, the client application is operably in communication with the distributed, sample processing units through communications to the server. Optionally, sample processing units are on data networks not local to the client application and the laboratory information system. Optionally, data pathway for sample processing data from the sample processing units to the client application comprises at least one wide area network. Optionally, the client application is operable on a mobile programmable device. Optionally, the client application is configured to extract data from a database on the server based on an event-driven methodology. Optionally, the sample processing data is analyzed at the laboratory to provide certified test results. Optionally, a private key/public key authentication is used to verify that devices are correctly paired Optionally, a private key/public key authentication is used for secure data transfer from a sending location to a desired recipient location. Optionally, sample processing data is decrypted by the client application. Optionally, the client application is operable to communicate commands regarding unit operation to the distributed, sample processing units associated with the laboratory. Optionally, the client application receives status information from the distributed, sample processing units associated with the laboratory. Optionally, laboratory information system comprises a computer processor configured to request and collect test results associated with the sample. Optionally, the sample is associated with the laboratory based on a sample identifier. Optionally, the client application informs the LIS of the distributed, sample processing units are operably in communication with the LIS. Optionally, the client application transforms sample processing data into test results in a file format acceptable by the LIS. Optionally, the client application comprises a personal computer with a touchscreen display or other user interface. Optionally, the client application comprises a software application running on a network connected cellular phone. Optionally, the client application is operable to monitor status of distributed, sample processing units in the field. Optionally, the distributed, sample processing units will not allow a sample to be inserted until the client application sends authorization. Optionally, the distributed, sample processing units will not allow a sample to be inserted until the client application sends authorization from certified personnel or other entity can authorize at least one of the sample processing units to run it. Optionally, access to the distributed, sample processing units is controlled remotely by a client application in the LIS. Optionally, the client application is operable to control access to the sample processing units prior to running the units, showing status of devices in action, and then how to process data/results after the system has run. Optionally, the client application is operable to show status of sample processing units in action. Optionally, the client application is operable to determine how to process data after one of the sample processing units has run its testing on the sample. Optionally, sample processing units receives a protocol specific for a sample it is processing and as long as it is network connected, the sample processing units download the protocol from a server different than the LIS.

In at least one exemplary embodiment described herein, a programmable device is provided comprising: a listener application configured for communicating with a server separated from the device by at least one wide area network, wherein said listener application configured for receiving sample processing data from a database with information for sample processing units associated with but operably separated from the listener application by at least one wide area network.

It should be understood that embodiments in this disclosure may be adapted to have one or more of the features described below. In one non-limiting example, the device further comprises a data verification application operable for pairing a biological sample processing unit and a laboratory information system at a destination laboratory, whereby data from the sample processing unit is unencrypted and saved into laboratory information system. Optionally, a command application configured to send commands to control operation of one or more of the sample processing units over a computer network. Optionally, the listener application also receives status information for sample processing units associated with this destination. Optionally, the device includes a data verification application operable for pairing a biological sample processing unit and a laboratory information system at a destination laboratory, whereby data from the sample processing unit is unencrypted and saved into laboratory information system. Optionally, the device includes a command application configured to send commands to control operation of one or more of the sample processing units over a computer network.

It should be understood that embodiments in this disclosure may be adapted to have one or more of the features described below. In one nonlimiting example, a device running a non-LIS client may be running a listener application. Optionally, a listener application for LIS is specific for certain types of sample processing devices. Optionally, an LIS interface device may be provided. Optionally, a method is provided for LIS interface with distributed sample processing units. Optionally, the method may include sending data to cloud, then having the data go to LIS. Optionally, there may be pairing authentication between a device in the field and the laboratory with the LIS. Optionally, the method may include sending data to LIS directly and pairing (w/authentication). Optionally, the LIS and systems with remote sample processing devices are on separate networks.

In at least one exemplary embodiment described herein, a method is provided for use with a laboratory information system (LIS) comprising: receiving sample data at the LIS from a database, wherein the sample data originates from at least one sample processing units at a physical location remote from a physical location of the LIS and wherein the database resident on a computing device at a location remote from the LIS, wherein the sample data traverses at least one data pathway through one or more wide area networks before reaching a data network comprising the LIS.

It should be understood that embodiments in this disclosure may be adapted to have one or more of the features described below. In one nonlimiting example, the method may further include authenticating sample data from the sample processing unit for at least two factors before processing the sample data for the database. Optionally, the method may further include processing the sample data to provided processed sample data that is stored in the database, wherein processed sample data is sent to the LIS. Optionally, the method may further include processing the sample data to provide interpretations of the sample data for storage in the database. Optionally, the method may further include using data from at least one assay calibrator to be one factor in authenticating sample data authenticity. Optionally, the method may further include using data from at least one control to be one factor in authenticating sample data authenticity, wherein the control comprises a component known to provide a pre-determined result. Optionally, the method may further include using a listener application operably in communication with the LIS and the database to receive the sample data from the database and then transfer to the LIS. Optionally, the method may further include using a listener application operably in communication with the LIS and the database to receive the sample data from the database and then transfer to the LIS.

In at least one exemplary embodiment described herein, a method is provided for use with a laboratory information system (LIS) comprising: receiving sample data at a listener application operably in communication with the LIS, wherein the sample data originates from at least one sample processing units at a physical location remote from a physical location of the LIS and wherein sample data is sent to a database resident on a computing device at a location remote from the LIS, and the sample data received by the listener application is sent from the database; wherein the sample data traverses at least one data pathway through one or more wide area networks before reaching a data network comprising the LIS.

It should be understood that embodiments in this disclosure may be adapted to have one or more of the features described below. In one nonlimiting example, the method comprises at least one technical feature from any of the prior described features. Optionally, the method comprises at least any two technical features from any of the prior described features. Optionally, the device comprises at least one technical feature from any of the prior described features. Optionally, the device comprises at least any two technical features from any of the prior described features. Optionally, the system comprises at least one technical feature from any of the prior described features. Optionally, the system comprises at least any two technical features from any of the prior described features.

This Summary is provided to introduce a selection of concepts in a simplified form that are further described below in the Detailed Description. This Summary is not intended to identify key features or essential features of the claimed subject matter, nor is it intended to be used to limit the scope of the claimed subject matter.

### BRIEF DESCRIPTION OF THE DRAWINGS

Figures 1A and 1B show embodiments of systems as described herein.
Figure 2 shows one example of a method according to at least one embodiment herein.
Figure 3 shows a schematic of data transfer according to at least one embodiment described herein.
Figure 4 shows a schematic of data transfer according to at least one embodiment described herein.
Figures 5A and 5B show a schematic of data transfer according to at least two embodiments described herein.
Figures 6 to 8 show various embodiments of a listener application operating on a hardware platform as described herein.
Figures 9 to 13 show schematics related various embodiments of listener applications and databases as described herein.
Figure 14 shows one non-limiting example of a plurality of sample processing units according to at least one embodiment herein.
Figure 15 shows one non-limiting example of computer architecture according to at least one embodiment herein.

### DESCRIPTION OF THE SPECIFIC EMBODIMENTS

It is to be understood that both the foregoing general description and the following detailed description are exemplary and explanatory only and are not restrictive of the invention, as claimed. It may be noted that, as used in the specification and the appended claims, the singular forms "a", "an" and "the" include plural referents unless the context clearly dictates otherwise. Thus, for example, reference to "a material" may include mixtures of materials, reference to "a compound" may include multiple compounds, and the like.

In this specification and in the claims which follow, reference will be made to a number of terms which shall be defined to have the following meanings:

"Optional" or "optionally" means that the subsequently described circumstance may or may not occur, so that the description includes instances where the circumstance occurs and instances where it does not. For example, if a device optionally contains a feature for a sample collection unit, this means that the sample collection unit may or may not be present, and, thus, the description includes both structures wherein a device possesses the sample collection unit and structures wherein sample collection unit is not present.

As used herein, the terms "substantial" means more than a minimal or insignificant amount; and "substantially" means more than a minimally or insignificantly. Thus, for example, the phrase "substantially different", as used herein, denotes a sufficiently high degree of difference between two numeric values such that one of skill in the art would consider the difference between the two values to be of statistical significance within the context of the characteristic measured by said values. Thus, the difference between two values that are substantially different from each other is typically greater than about 10%, and may be greater than about 20%, preferably greater than about 30%, preferably greater than about 40%, preferably greater than about 50% as a function of the reference value or comparator value.

As used herein, a "sample" may be but is not limited to a blood sample, or a portion of a blood sample, may be of any suitable size or volume, and is preferably of small size or volume. In some embodiments of the assays and methods disclosed herein, measurements may be made using a small volume blood sample, or no more than a small volume portion of a blood sample, where a small volume comprises no more than about 5 mL; or comprises no more than about 3 mL; or comprises no more than about 2 mL; or comprises no more than about 1 mL; or comprises no more than about 500 µL; or comprises no more than about 250 µL; or comprises no more than about 100 µL; or comprises no more than about 75 µL; or comprises no more than about 50 µL; or comprises no more than about 35 µL; or comprises no more than about 25 µL; or comprises no more than about 20 µL; or comprises no more than about 15 µL; or comprises no more than about 10 µL; or comprises no more than about 8 µL; or comprises no more than about 6 µL; or comprises no more than about 5 µL; or comprises no more than about 4 µL; or comprises no more than about 3 µL; or comprises no more than about 2 µL; or comprises no more than about 1 µL; or comprises no more than about 0.8 µL; or comprises no more than about 0.5 µL; or comprises no more than about 0.3 µL; or comprises no more than about 0.2 µL; or comprises no more than about 0.1 µL; or comprises no more than about 0.05 µL; or comprises no more than about 0.01 µL.

As used herein, the term "point of service location" may include locations where a subject may receive a service (e.g. testing, monitoring, treatment, diagnosis, guidance, sample collection, ID verification, medical services, non-medical services, etc.), and may include, without limitation, a subject's home, a subject's business, the location of a healthcare provider (e.g., doctor), hospitals, emergency rooms, operating rooms, clinics, health care professionals' offices, laboratories, retailers [e.g. pharmacies (e.g., retail pharmacy, clinical pharmacy, hospital pharmacy), drugstores, supermarkets, grocers, etc.], transportation vehicles (e.g. car, boat, truck, bus, airplane, motorcycle, ambulance, mobile unit, fire engine/truck, emergency vehicle, law enforcement vehicle, police car, or other vehicle configured to transport a subject from one point to another, etc.), traveling medical care units, mobile units, schools, day-care centers, security screening locations, combat locations, health assisted living residences, government offices, office buildings, tents, bodily fluid sample acquisition sites (e.g. blood collection centers), sites at or near an entrance to a location that a subject may wish to access, sites on or near a device that a subject may wish to access (e.g., the location of a computer if the subject wishes to access the computer), a location where a sample processing device receives a sample, or any other point of service location described elsewhere herein.

A "cluster" is a system in which multiple database servers ("instances") have access to the same database. A database to which the multiple instances have access is referred to herein as a "cluster database." The persistent storage that stores a cluster database is accessible by all instances in the cluster. Typical database objects such as persistent tables, packages and procedures will be accessible from any instance of the cluster database. In a cluster database, one or more instances of a certain class of objects may be stored on one or more database instances in a private area accessible only to that instance, e.g. in its volatile memory. For example, in a database cluster containing instances I1, 12, and 13, a particular object, O1, may be stored on I1 and 13 in their respective volatile memory, but may not be stored on 12. Connecting to such objects in a cluster environment presents a unique challenge because the information destined for the particular object in a cluster database does not merely have to be delivered to the right database, but has to be delivered to the right instance (an instance containing the target object). To ensure that information is delivered to the correct database instance, database links could be allocated on an instance-by-instance basis.

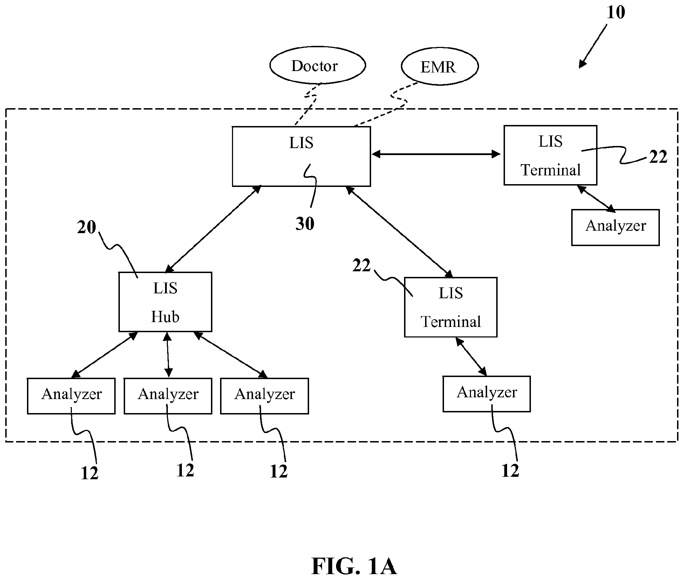

Referring now to Figure 1A, when a plurality of biological sample analyzers 12 are in a laboratory 10, there is typically at least one connectivity hub 20 such as but not limited to a data connectivity hub such as a USB hub, wifi hub, or other data protocol hub that physically connects the sample analyzers 12 to the LIS 30. In some cases, there is a terminal 22 (instead of a USB hub) that connects to the multiple sample analyzers 12. Optionally, there may be multiple terminals 22, multiple hubs 20, and/or multiple sets of analyzers 12. There can be multiple computers, terminals, or servers that are brokers that run middleware to send the information to LIS 30. These computers, terminals, or servers are also running the LIS software, which allows the data to be sent to a database in the LIS 30.

For this system to work, the LIS 30 typically has sufficient intelligence such as but not limited to one or more programmable processors so that it knows which sample processing devices and/or analytical devices are operably coupled to the system. This can be challenging since different sample processing and/or analytical devices are often made by different manufacturers. Most of the analyzers 12 generate data in a common format such as but not limited to a comma separate value (CSV) file. In one non-limiting example, LIS 30 can communicate with the devices using a common programming interface that the industry has informally agreed upon. The LIS 30 can request the analyzer 12 to send data, which is typically in the common format such as but not limited to the CSV file. That file format may be different for each analyzer 12. One or more adapters (software and/or hardware) take data from an analyzer 12, convert it to a common format, and upload it in common format in the LIS 30. Optionally, one or more adapters (software and/or hardware) take data from a analyzer 12, convert it to one of a plurality of formats recognized by the LIS 30, and upload it in the format in the LIS 30.

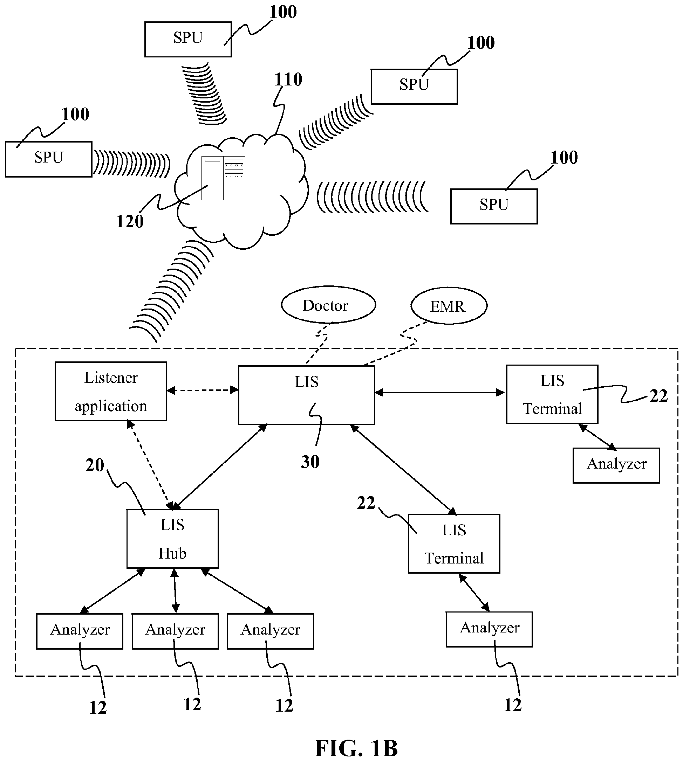

Referring now to Figure 1B, in at least some of the embodiments herein, because of on-line connectivity of sample processing units, the embodiments herein can do something very unique. In one embodiment, there may be a network connected broker and/or listener application 50 coupled to the LIS system 30. In one non-limiting example, this broker and/or listener application 50 communicates through the network, wired, wirelessly, or online to analyzers 12 (local or distant). Optionally, data and/or other information may be received by the broker and/or listener application 50 which then transmits the data and/or other information to LIS 30. Optionally, in some embodiments, the broker and/or listener application 50 can process the data and/or other information to be in a format accepted by the LIS 30. Optionally, in some embodiments, the broker and/or listener application 50 can process the data and/or other information to be sorted, analyzed, or otherwise handled prior to transmission to the LIS 30.

The traditional configuration of clinical laboratories is limited to embodiments where there is a local area connection, typically physical. In some cases, the connection can be Ethernet (device to PC may be a local protocol such as USB or optionally from PC to LIS by Ethernet). It should be understood that for at least one or more embodiments herein, data is not limited to data received from physically local sample processing or analytical devices. Internet connectivity by wide area network (including GSM, CDMA, Satellite, etc...) or other network connection allows for data to be received from distant, sample processing devices.

### Data Formatting

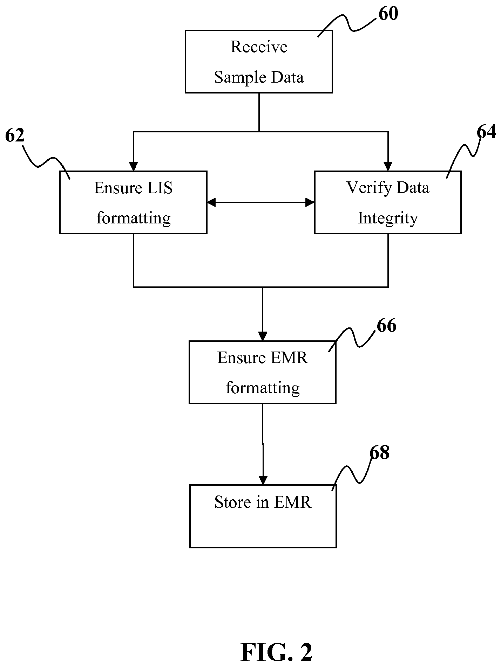

Referring now to Figure 2, it should also be understood that some embodiments may use the interface into the LIS 30 as a method to reformat or alter format of data received from a remote device so that data from that remote device can be stored in a medical database such as but not limited to an EMR that may be associated with LIS 30. As seen in this embodiment of Figure 2, the data may be received at step 60. By way of example and not limitation, this data can be through the listener and/or broker application 50. Optionally, it can be through another interface into the LIS 30. As shown in step 62, there may be a data formatting step where one or more steps are taken to ensure by way of formatting or other processing that data is in a format acceptable to the LIS 30. This may be a verification step followed by a processing step if it is determined that data is not in the desired format(s). Optionally, some embodiments may proceed directly to a data format processing step without or concurrently with the data format verification step. Some embodiments of LIS 30 accept primarily CSV file format. It is advantageous to select a format that is commonly used by many of the LIS laboratory systems. This common format used by many of these systems allows for the data to be more easily processed for storage into a second database system that is characterized as having more possible data formats that are to be accounted for than the number of formats associated with the LIS system. Handling more data formats makes for a more cumbersome solution in terms of having sufficient data processing capability to take one format and processing it into one of the formats acceptable by that particular EMR or other second database system. By interfacing at a location in the overall infrastructure where there are few formats to have to prepare for or accommodate, this can simplify the process of getting data into the two or more healthcare database systems.

By way of non-limiting example, once data has been entered into the LIS 30 and/or before it is entered into the LIS 30, there may be a data integrity verification step 64. In some embodiments, this can occur in a device or application that is part of the infrastructure that is local to the laboratory and/or as part of the area network associated with the LIS 30, connectable through a non-WAN network. This allows data from the remote device(s) 100 to be verified when it is being processed after it has been sent from the cloud to be entered into the LIS 30.

By way of non-limiting example, once data has been entered into the LIS 30, it can then be processed to be entered in the EMR system. Often, such an interface already exists in legacy deployments where the data in the LIS can be released and stored in the EMR. Thus, in at least one of the embodiments herein, instead of directly interfacing with the EMR, the circuitous path of first entering data into the LIS can simplify the implementation of getting data from a remote device 100 into the EMR system. Ensuring that the data is in an EMR-acceptable format can include but is not limited to at least one verification step followed by at least one processing step if it is determined that data is not in the desired format(s). Optionally, some embodiments may proceed directly to a data format processing step without or concurrently with the data format verification step. Some embodiments may store data in the EMR and then proceed with or concurrently with the ensuring step. Optionally, some systems may opt not to store data in the EMR until there has been data that is ensured to be in the desired format for the second healthcare database system. Although EMR is listed herein as the other database, it should be understood that another type of healthcare database currently known or developed in the future may be the database for which data from the LIS or similar first system can be formatted or otherwise processed for compatibility.

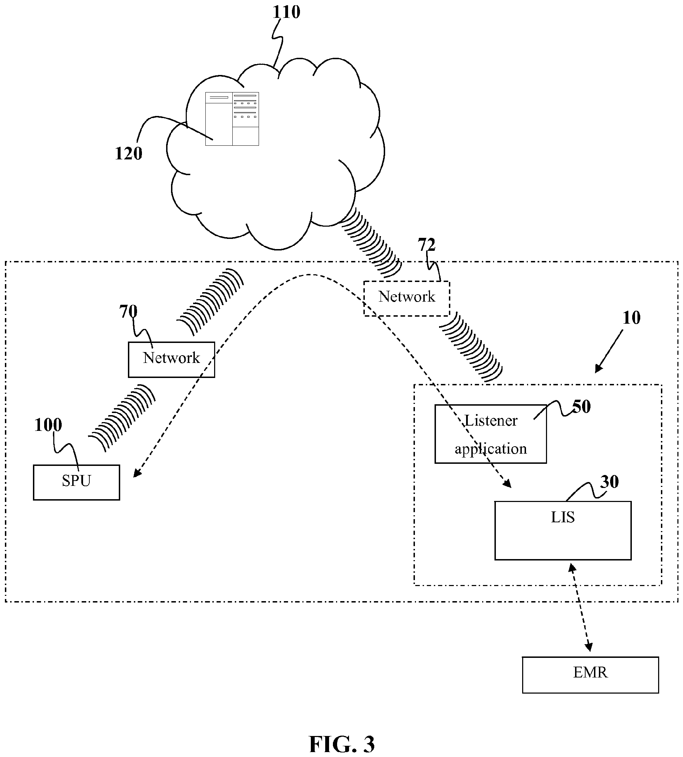

Referring now to Figure 3, at least one exemplary embodiment of a system for use with at least one method herein will now be described. Figure 3 shows that in this embodiment, information can be sent from the device 100 through a network 70 to the cloud 110. By way of non-limiting example, the cloud 110 comprises one or more servers 120 in one or more data networks. Server 120 may be a cluster of servers. In one non-limiting example, a database on one or more of the servers 120 may be a cluster database. By way of non-limiting example, the cloud 110 comprises one or more computing devices in communication with one or more data networks. As seen in Figure 3, data is then sent from the cloud 110 through a network 72 to the physical laboratory 10 with co-located or locally connected LIS 30 therein.

As seen in Figure 3 for some embodiments herein, as data is sent to the cloud 110, the metadata in the file may be corrupted or not provide desired information regarding when test was taken. Some embodiments herein may opt not to use any of the metadata associated with the data. Optionally, some embodiments may extract metadata at the device 100 and include it as part of the data such as but not limited to a value of one or more the data fields that are transmitted, instead of residing in the background as metadata. Optionally, the harvesting of the metadata can occur in the cloud 110. Optionally, it may continue to be part of the metadata of the file or it can be incorporated into one or more the data fields that are transmitted onward to the laboratory 10 with the LIS 30.

It should be understood that the networks 70 and/or 72 may be Wide-Area Networks (WAN). In some embodiments, the internet can be considered a WAN. WANs are commonly connected either through the Internet or special arrangements made with phone companies or other service providers. A WAN is different from a MAN because of the distance between each of the networks. In a WAN, one network may be anywhere from several hundred miles away, to across the globe in a different country.

Figure 3 shows that once data has been incorporated into the LIS 30, there may be a further step wherein data can be transferred to and entered into an EMR or vice versa. For systems with LIS and EMR already implemented, this connectivity may already exist. Thus, adding remote sample processing devices 100 (or analytical devices) that interface with the LIS 30 can expedite this implementation as the number of data formats that are to be accounted for are much smaller for systems that already have an LIS 30. This provides an expedited path to implementation for data entry into the EMR by way of another system such as the LIS 30. Of course other systems that communicate with the EMR and that also provide a facilitated interface such as but not limited to having fewer data formats or the like can also be used as a data preparation pathway to enter information into the LIS 30.

### Oversight of Distributed Laboratory Equipment

One advantage of some embodiments of systems described herein is that even though these remote devices 100 are in the field as sample processing units in locations that may be physically remote from the clinical laboratory or other authorized analysis facility, they may still be functionally part of a laboratory and functionally linked to the LIS 30. Wherever the devices 100 are located, such as but not limited to being physically separate from the clinical laboratory in a different building or physically located hundreds of miles away, data is still coming into the laboratory system and into LIS 30. In one non-limiting embodiment, the results are not displayed anywhere except in the laboratory or to laboratory personnel associated with the LIS 30. In one embodiment, the devices 100 in the field are sample processing units that do not display results at the device 100 processing the sample(s). In one such embodiment, data is transmitted to an LIS 30 (not directly to an EMR) for processing and/or certification.

In a further embodiment, the broker and/or listener application 50 can be operated on a device such as but not limited to a personal computer with a touchscreen display or other user interface. It should be understood that other embodiments may use tablet computers, mobile phones, wearable computers, smart watches, or other devices to provide control of the devices in the field. This allows the laboratory director or other authorized personnel to monitor status of device(s) 100 in the field, particularly and/or only those remote devices 100 associated with the laboratory. Optionally, the authorized personnel can also see what is being run in terms of assays and/or samples, status of the components in the remote sample processing unit, and where they are in the world. Optionally, in addition to or in place of receiving data from devices 100 or from monitoring their status, some embodiments can also send commands to control devices 100 such as but not limited to sample processing units. Thus, before a sample is inserted, the laboratory, through its authorized personnel or other entity, can authorize the device 100 to run it. For some embodiments, one can also only open the drawer, access cover, or other sample loading assembly on for those tests or processes with lab director approval to be run on the device 100. Thus, access to the device 100 can be controlled remotely.

In a hospital setting, the devices 100 can be also be controlled remotely. Thus, some can provide two-way communication to allow a laboratory director or other authorized personnel to control infrastructure, including those devices that may be remote from the laboratory.

In some embodiments, the broker and/or listener application 50 can be a mobile phone application run on a mobile or cellular platform device. Optionally, in other embodiments, the broker and/or listener application 50 can operate on other device platforms. As previously discussed, the hardware for the broker and/or listener application 50 does not need to be a traditional personal computer. In this non-limiting example, the phone with the broker and/or listener application 50 could be connected by USB or other data connection to the LIS. Because the phone or device may be always or substantially always connected (whether by wifi, cellular network, or other current or future data connectivity technique), the phone can be used to control the infrastructure of remote devices. This can be an LIS adapter to the entire LIS network. The system may be about how to control access to the SPU's prior to running the device, showing status of devices in action, and then how to process data/results after the system has run.

In one embodiment, all of the protocols, such as but not limited to the assay protocols for sample processing at the devices 100, may still come for a different source (not the laboratory) such as but not limited to a remote server or computer operated by a third party not directly operating the laboratory, such as but not limited to the device manufacturer or a service provider.

With regards to the flow of information between the devices 100 and the LIS, most of the communication is from the devices 100 to the LIS 30. Typically, there is less communication from the LIS 30 to the devices 100 such as the sample processing unit. When pulling up the results, the barcodes are typically barcodes that LIS 30 recognizes. In most embodiments, it is LIS 30 that pulls data from the sample processing devices 100. It should be understood that in some embodiments, this is an indirect connection wherein data from the sample processing device 100 is sent to an intermediate device such as but not limited to a computing device having a database therein, and then having the LIS 30 or an application in communication with the LIS 30 retrieve the desired information from the intermediate device. In at least some but not all embodiments, the device 100 rarely pushes data to the LIS 30 prior to receiving an LIS request. LIS can also react when an event is noted in the device 100 and then poll the device when LIS 30 needs the data. Data can also be deleted from device 100 after it is pulled into LIS 30. LIS systems can have adapters written to allow different devices 100 to communicate with the LIS 30.

It may be desirable to have the SPUs close the patient, but the results should be displayed at least in the laboratory so that the LIS system and lab director or other authorized personnel can review the data and/or results from these remote devices.

In at least some embodiments herein, the devices 100 sends sample processing data to the cloud 110. By way of non-limiting example, the cloud 110 may be one or more servers 120 that form the cloud 110. Communication from the devices 100 can be by way of one or more communication protocols. Some may use a channel access method selected from frequency division multiple access (FDMA), wavelength division multiple access (WDMA), orthogonal frequency division multiple access (OFDMA), based on Orthogonal, frequency-division multiplexing (OFDM), single-carrier FDMA (SC-FDMA) (or linearly-precoded OFDMA (LP-OFDMA)), time-division multiple access (TDMA), code division multiple access (CDMA) (or spread spectrum multiple access (SSMA)), direct-sequence CDMA (DS-CDMA), frequency-hopping CDMA (FH-CDMA), orthogonal frequency-hopping multiple access (OFHMA), multi-carrier code division multiple access (MC-CDMA), space division multiple access (SDMA), packet mode channel access methods (e.g., contention based random multiple access methods), duplexing methods (e.g., time division duplex (TDD), frequency division duplex (FDD)), global system for mobile communications (GSM), GSM with GPRS packet, bluetooth packet mode communication, IEEE 802.11b wireless local area networks (WLAN's), high performance radio local area network (HIPERLAN/2) wireless networks, and G.hn. A wireless provider may be configured for second-generation cellular wireless telephone technology (2G), third generation mobile telecommunications (3G), fourth generation cellular wireless standards (4G) or LTE Advanced (LTE) communication standard. Optionally, some embodiments may use network connectivity methods as described in U.S. Patent Application Ser. No. 13/784,814 filed March 4, 2013.

Thus, sample processing data and/or sometimes test results go to a cloud location 110. Optionally, some may only send data that will be further processed into analytical results at the laboratory such as but not limited to embodiments described in U.S. Patent Application Ser. No. 61/766,095 filed February 18, 2013 and fully incorporated herein by reference for all purposes. In this embodiment, the listener application 50 receives that data from the cloud 110 for the five devices 100 for a laboratory that just finished processing their samples. The results are loaded to the cloud 110 and the listener application 50 faithfully notifies LIS 30 when result is received in the cloud 110 for any of the devices in all of the infrastructure that are functionally part of that laboratory. The listener application 50 can retrieve data from any of the analyzers and/or sample processing units in communication with the cloud 110. Optionally, the listener application 50 is a proxy for all of the devices 100 such as but not limited to the sample process units in the distributed infrastructure. In this embodiment, the proxy is listening to all of the infrastructure of devices 100 through the cloud, wireless, or other network connectivity or information communication technique. Integration with laboratory information system 30 creates one embodiment of a system that can retrieve information from different analytical devices using any connectivity technique (wireless, etc...). By way of non-limiting example, this integration can be achieved in part by the use of one or more listener and/or broker applications 50. By having such a proxy, the devices 100 that may be but are not limited to sample processing units not need be on the same network, can be remote, etc... In such an embodiment, one can connect this adapter/listener/proxy to an LIS 30 and integration of the device 100 to a system such as but not limited to an EMR is also done.

It should be understood that often, the local protocol from the LIS 30 to a local analyzer in the laboratory is typically a vestige of a device requirement that has the devices in the laboratory. Typically, the reason these local analyzers are designed in this manner relates to the requirement of running in a Clinical Laboratory Improvement Amendments (CLIA) certified laboratory. Although some systems may send data wirelessly to EMR, they do not send directly to LIS 30. It should also be understood that the file format for the LIS 30 is more commonly based on variations of one format, whereas the file format for an EMR systems generally has no linkage or relation to a common format.

In one embodiment of a system described herein, CLIA or other laboratory certification may involve having the oversight issue addressed by one or more solutions. In one non-limiting example, information about sample testing does not go to a physician until it goes to a laboratory managed director or authorized personnel as part of the LIS 30; laboratory managed director or authorized personnel has total control of the quality of this information such as but not limited to all the controls, the calibrators, duplicates/triplicates, and the performance of the device are fed to the laboratory managed director or authorized personnel who can look at the device information including performance information remotely and once they are satisfied they can green-light sending the data/result to LIS 30. Optionally, the data is sent directly to LIS 30, but laboratory managed director or authorized personnel can go see the individual machine performance if the data to LIS 30 triggers certain flag. In this non-limiting example, the laboratory managed director or authorized personnel can touch-click expand, see the quality of the data, performance, and/or replicates to verify if they trust the data.

Even without oversight for waived devices, there are no CLIA waived devices that plug directly to or sends data directly into LIS 30. In the context herein, waived devices are devices that provide the results to medical personnel and those personnel will process the results, typically without benefit of being able to ascertain for themselves the integrity of the entire testing process and/or hardware. For example, an iSTAT monitor directly communicates with the EMR to upload data. It sends its test data directly to the EMR system. By contrast, data does not go to the LIS until the lab director approves sending the results to LIS.

It should be understood that a laboratory director or authorized personnel can take information that has come in from a waived device and enter into LIS 30 if they can trust the results. One scenario where this may occur can be in situations wherein because of other multiple previous results being acceptable, there is a historical record of accurate results. When there is no reason to suspect that results are not ok, then the laboratory director or authorized personnel can deem that they can trust the results based on this historical record. Additionally or alternatively, waived device in the laboratory can also be deemed to provide trusted results if, for example, the lab director or other authorized personnel runs controls on the waived device and/or provides some other method to verify integrity of the testing. Optionally, laboratory director or authorized personnel can check the history of a waived device and see if he or she wants to send on the results to the LIS based in part on performance history. It should be understood that the laboratory director is responsible for the quality of the results. As part of this, one can check to see when controls were on. This may provide the basis to give approval for a doctor to rely on the results. Based on the foregoing, some may convert results from waive device to results from a CLIA certified environment, even if the device is waived and remote. For at least some embodiments herein, the advantage here is that analytical and/or sample processing device can be anywhere in the world but laboratory director can trust it based on knowledge about the device and its recent performance history. Optionally, some embodiments may configure the remote device to have limited local user control of the device. Additionally, the laboratory director can push quality control (QC) out to the analytical or sample processing device to tell it to run calibrator(s) or to shut it down until someone runs a calibrator (taking the device off-line) until a control cartridge and/or control protocol is run.

In one embodiment herein, the system can provide detailed monitoring of the remote device 100 and the ability for the laboratory director or other personnel to control device. Controlling the device may including being able to see status, shut down the device, open sample loading door, limit device access, etc....

In one embodiment herein, the system does not require a physical wired connection for the device 100 to send data to the LIS 30.

In one embodiment herein, the system can provide full control of the device 100. The system in this example is configured to look at device quality through monitor and can have every detail that one would have as a manufacturer. Data about the machine. Policy.

In one embodiment herein, the device 100 has a connection to LIS 30 that is wireless. Optionally, some may view this as a brokerless LIS system. In the embodiment, the cloud 110 is the broker. Optionally, there is a pairing mechanism that associates certain machines or servers in the cloud with certain listener applications 50. Optionally, an administrator can set which machines or servers are in the environment. The system can also search the network to see which machines or servers are in the environment. If the device is not on the same LAN, it is still accessible on WAN. This listener application 50 is only listening for its designated set of machines.

In some embodiments, the system can be configured such as the system with the LIS 30 will be there to receive results from a reference laboratory. A reference laboratory may be one that performs sample testing but is not the laboratory that reports out the results to the patient and/or physician. In this non-limiting example, the system may have one or more sample processing devices 100 that report data to a reference laboratory that finalizes the results and sends the data to the receiving laboratory, or sends the receiving laboratory the raw sample data through a pathways such as through a gateway including but not limited to a broker application and/or listener application 50. Service provided by a reference laboratory allows for greater capacity for the receiving laboratory to process samples and send out test results while still maintaining a seamless interaction between the laboratory and the patient or physician. Even if one laboratory such as a reference laboratory has looked at the test results, the receiving laboratory still reviews and signs off on the test results. The results may then be relayed as results certified by the receiving laboratory. By way of example and not limitation, three scenarios include, but are not limited to: analyzer device to LIS, reference lab to another lab, or lab providing service directly to doctor.

Optionally, there can be data ports "opened" on the device so that directions, instructions, or other information can be sent to the device 100 not just from any touchscreen or local input device, but also from the laboratory director or other authorized personnel who may be remotely located.

It should be understood that at least some embodiments herein provide for wireless, distributed laboratory infrastructure. At least some of these embodiments may use fully distributed stand-alone devices communicating information to and from a secure LIS infrastructure. Optionally, the devices are stand-alone sample processing devices. Optionally, the devices are stand-alone analytical devices. In some embodiments, they do not require a wired USB or PC interconnection.

Optionally, at least some of the embodiments herein provide for authentication of identities for the device 100 and the LIS 30. As discussed, the device 100 may be a stand-alone analyzer/medical device. Optionally, at least some of the embodiments herein provide for secure, reliable communication with at least one designated LIS 30. Optionally, at least some of the embodiments herein provide for secure, reliable communication with at least one designated LIS 30 over the cloud. Optionally, at least some of the embodiments herein provide for secure, reliable communication with at least one designated LIS 30 through two-way communication with the LIS. Optionally, at least some embodiments herein provide for reliable "pairing" with LIS over internet. Optionally, at least some embodiments herein provide for device authentication by geolocation.

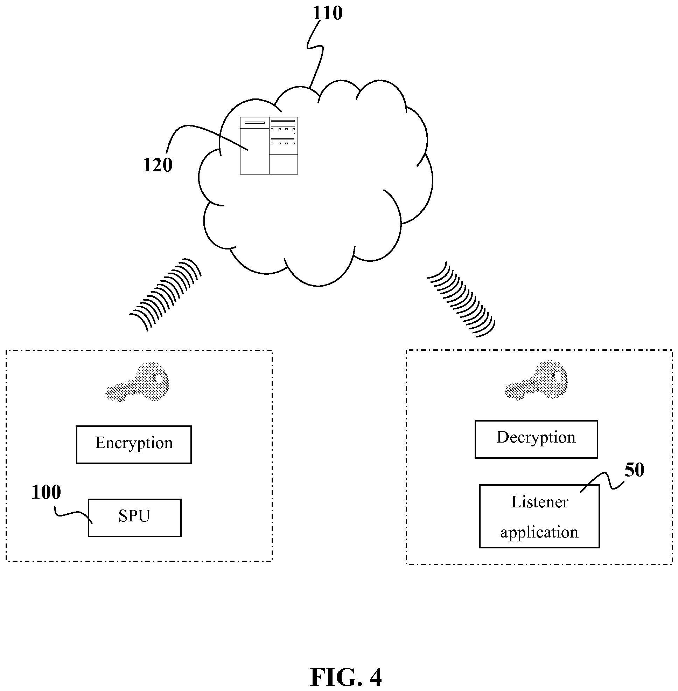

Referring now to Figure 4, it should be understood that data communication between the LIS 30 and the device 100 can be by way of secure data communication such as but not limited to encryption that secures data. Optionally, some embodiments may also use secure pairing such that the data is only sent to the right designation and/or is only pulled from the right machines. In one embodiment, this reliable pairing occurs with LIS 30 over the internet. By way of non-limiting example, a laboratory director or other authorized personnel who installs this can retrieve laboratory credentials and remote device credentials and confirm that each is correct before making a pairing between a device 100 and the LIS 30, which may be through a pairing with the listener application 50. Optionally, the system can be configured to periodically check for a test of the pairing or to test device calibration. Optionally, these can involve using private key/public key technique. In at least one embodiment, all of the foregoing could use certificates authentication or electronic authentication.

Secure authentication of entities is currently something not used in laboratory analyzers. In at least one embodiment herein, the analytical device or sample processing device 30 authenticates itself when it connects to LIS. Analytical devices do not do that today. Optionally, the analytical device or sample processing device authenticates itself when it connects to a server or other destination in the cloud where data is being sent.

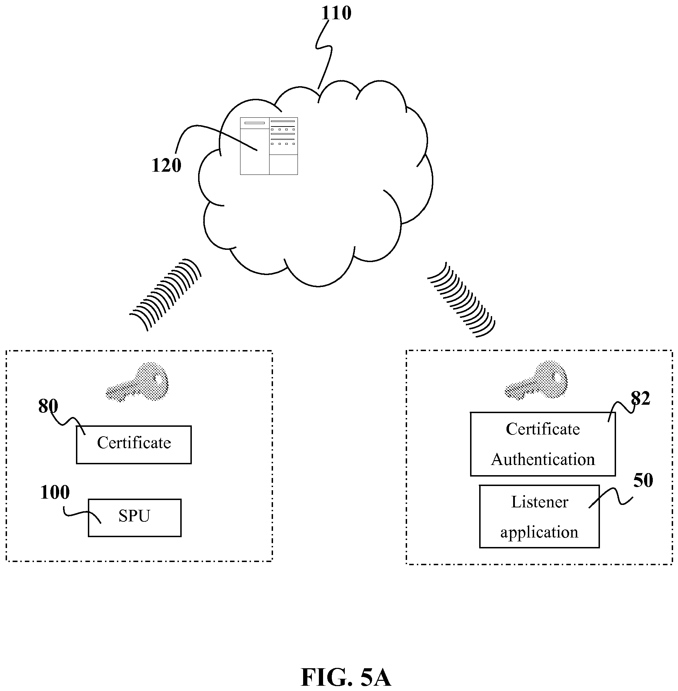

Referring now to Figure 5A, when in a distributed configuration, it may be desirable that the entity receiving the data be authorized to receive the data. This may be particularly true when data is being transmitted over the cloud, such as but not limited to being over a WAN and not just local, physical network connectivity. In some situations, being a secured device may not be enough. By way of non-limiting example, one could send information to the database in the cloud, but the system may also query and/or verify whether it should also be sending the data onward to final destination such as a destination laboratory. This can occur when the identity of the sender, intermediary, and/or final destination are all verified. Optionally, device 100 and listener application 50 can both have certificates 80 and 82, wherein matchmaking can occur in the cloud 110, at a server 120, or at a manufacturer's facility. If an entity does not have the certificate, that entity cannot establish a pair and/or cannot decrypt the data. One can also send messaging or pre-set instructions to expire a certificate if a certain condition occurs or too much time has passed.

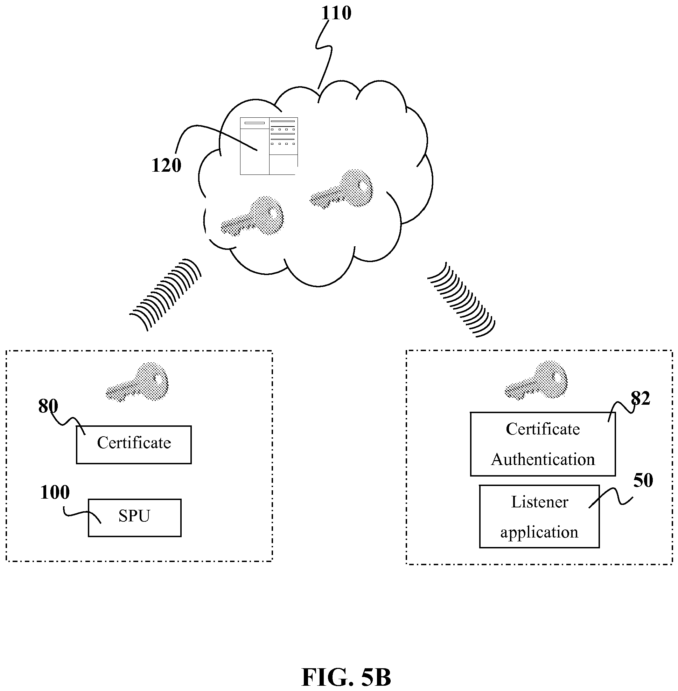

Optionally as seen in Figure 5B, some embodiments may have a decryption key for data received from SPU 100 and an encryption key for sending data to the listener application 50. This set of keys allows for separate encryption and decryption for a) communication from the SPI 100 to at least one server 120 in the cloud and for b) communication from the at least one server120 in the cloud to the listener application 50.

It should be understood that in some embodiments, the analytical device or a sample processing device are designed to run in only certain environments to lock it down in terms of system security. By way of non-limiting example, some embodiments may be configured to run in only one geolocation and/or in a set of locations set by an authorized entity such as an authorized user or the laboratory. Some embodiments can also improve test integrity by securing identity through geolocation of the device 100 and/or LIS 30 and then optionally, querying whether the location of device 100 is acceptable for performing sample processing. Geolocation may occur by way of GPS, internet IP address, connection to wireless access point, cellular data tower(s), or other techniques known or developed in the future. Some embodiments may include hardware onboard device 100 for geolocation sensing purposes.

At least one or more of the embodiments herein are useful in the field(s) of laboratory information systems, secure medical data, integration with live information system, or the like. Traditional laboratory automation systems typically control only the machine, but not how to handle the test data.

Because the system is distributed, at least some embodiments of the testing infrastructure described herein can be assigned in a dedicated and/or in an on-demand manner to hospital laboratory, health plan laboratory, or the like. For example, all retail locations can have oversight from Johns Hopkins University Hospital laboratory or the like for branding and quality perspective. In this non-limiting example, Hopkins can provide the medical care. The device manufacturer of device 100 can provide the technology platform. In this non-limiting example, once the device manufacturer gives results to LIS, the manufacturer is no longer directly involved in the care of that patient for that particular test. The laboratory responsible for branding and quality perspective will have the direct patient and/or health provider contact.

In such a distributed laboratory system, this configuration enables the laboratory director and/or authorized personnel to focus on the test results and analysis and less on keeping the equipment and everything else up and running. In at least one embodiment described herein, an authorized laboratory personnel can see device operation and/test status over a mobile device such as but not limited to a tablet computer, a wearable computing device, a watch computing device, or other computing device. There may be apps or operating system specific software that can be used. This allows for management of all of the devices 100 through a single computing device. Optionally, multiple devices 100 are managed through a single computing device. Optionally, multiple devices 100 are managed through multiple computing devices. Optionally, at least one device 100 is managed through multiple computing devices. Optionally, the computing device interfaces with a server that then manages one or more of the devices 100. Optionally, the computing device interfaces with one or more intermediary devices or services who then directly or indirectly manages one or more of the devices 100.

It should also be understood that some embodiments can assign certain modules within a device 100 or system of devices 100 associated in a pre-set or in an on-demand manner with a laboratory. By way of non-limiting example, at a patient service center (PSC), there may be multiple hospitals and/or laboratories being serviced by that PSC. For example, a physician may orders laboratory tests for a patient in an EMR. The physician or nurse may instruct the patient to go to that hospital's laboratory. But now, with a distributed laboratory infrastructure, one can transmit sample data from that distributed location to the correct laboratory. To the physician, it looks like the patient did their test at the hospital's laboratory, even if the actual sample was taken and/or processed at a remote site, due at least in part to the integration with that hospital's LIS 30. Although this example is described in the context of the hospital being the designated laboratory, others where the designated laboratory may be a physician group's laboratory, a health plan's laboratory, or other entities laboratory can also be applicable. This paradigm is not limited to a hospital laboratory. Laboratories with other associations are not excluded.

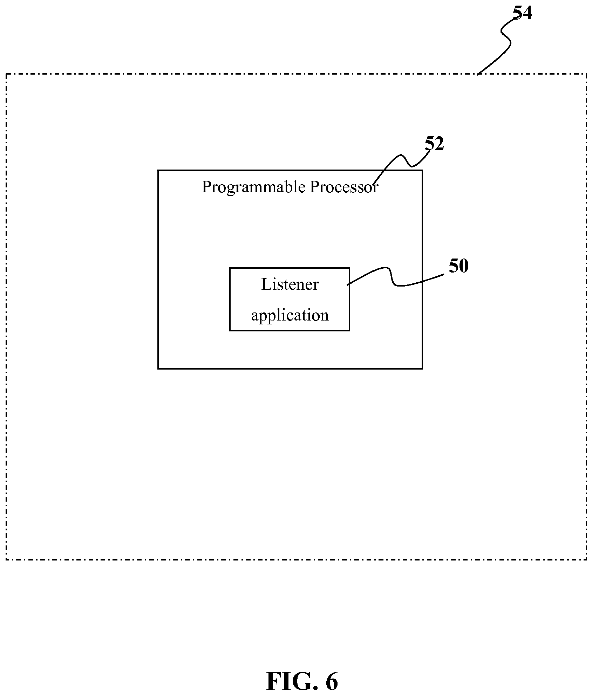

Referring now to Figure 6, it should be understood that in one embodiment, the listener application 50 may be software, an app, or other instruction that is used in conjunction with a programmable processor 52. Both the listener application 50 and the programmable processor 52 can be part of a larger instrument 54 such as but not limited to a computer, analyzer, handheld analyzer, mobile computing device, tablet computer, a cellular phone, a smart phone, a watch computing device, or the like.

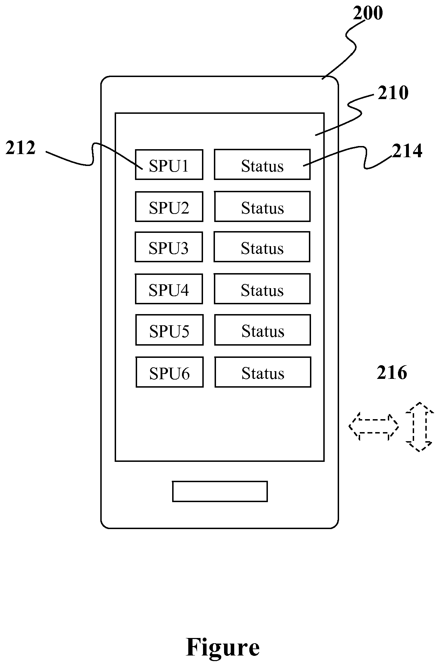

Referring now to Figure 7, it should be understood that in one embodiment, the instrument 54 may be a mobile computing device 200. In this embodiment, the screen 210 of the mobile computing device 200 can be configured to display a variety of different information about the sample processing units. Figure 7 shows that screen 210 in the embodiment shows that multiple sample processing units (SPU's) are shown as graphically representations 212 on the screen and that there is a status indicator 214 next each of the SPU graphical representations. It should be understood that the status indicator 214 can be but it not limited to colored bar (red, yellow, green, or the like), a text indicator (ok, alert, error, or the like), and/or a graphic or image showing status. In some embodiments, the screen 210 may display status for a single SPU 212, for multiple SPUs, or for multiple SPUs over several screens. In this manner, the screen can be swiped and/or scrolled (vertically or horizontally) as indicated by arrows 216 to reveal other information such as but not limited to status on more SPUs, for additional details on status, or the like.

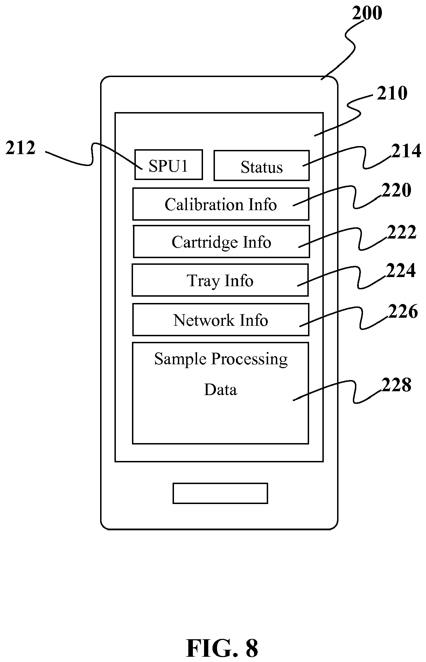

Referring now to Figure 8, it should be understood that in another embodiment, , the screen 210 of the mobile computing device 200 can be configured to display a variety of different information about just one sample processing unit. This embodiment can be the result of there being only a single SPU in communication with the computing device 200. Optionally, there may be a pinch-to-zoom, touch activated, gesture activated, voice activated, or other action taken by the user to expand information about an SPU 212. As seen in Figure 8, the status indicator 214 displayed on a screen shown in Figure 7 can continue to be part of the display shown in Figure 8. Figure 8 also shows that in this embodiment, the screen may also display calibration information 220 regarding the device, assay, or other aspect of the device or test.

Figure 8 also shows that other information about the SPU 212, the assay it is running, or the like can also be displayed on the screen. It should be understood that, in some embodiments, the information on the screen is not fixed and can be customized. For example, the screen 210 can display cartridge info 222, tray info 224, network info 226, and/or sample processing data 228. By way of non-limiting example, cartridge info 222 can relate to information about temperature of the cartridge, reagents in the cartridge, the identification or type of the cartridge, or other information. Optionally, tray info 224 can relate to information about whether the tray and/or access door for loading a sample on a cartridge or the like into the sample processing unit is open or closed. Optionally, network info 226 can relate to information about the status of data connectivity from the cloud to the sample processing unit, from the listener application 50 to the sample processing unit, or the like. Optionally, sample processing data 228 can relate to information about the sample being processed. Optionally, sample processing data 228 can relate to information about a calibrator if the unit is undergoing maintenance or other upkeep.

### Listener Application

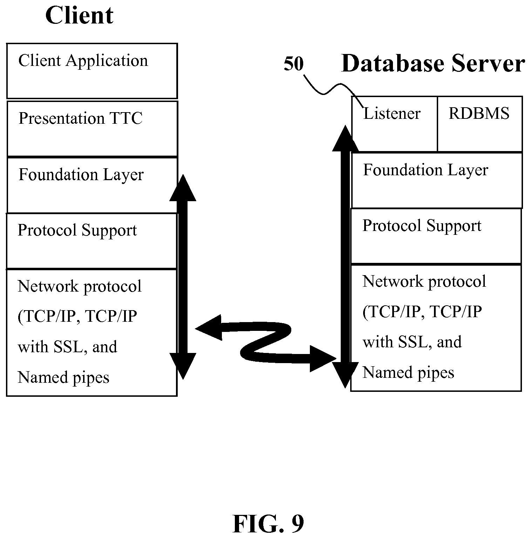

Referring now to Figure 9, at least some embodiments of a listener application 50 will now be described. In one embodiment, the listener application 50 may pull messages from queue and then update a database. Optionally, the application 50 is responsible for reading all inbound data. Optionally, it can send an ACK/NACK back to sender of the message. Optionally, the application 50 may be a listener such as asyn or sync listener. It may be designed to allow unattended updates of local and remote systems in a safe manner, with the ability to roll back an update. Optionally, the listener application 50 may be an event listener, wherein actions by the listener are event driven. In one embodiment, the listener application 50 may be a separate process that runs on the database server computer. Optionally, the listener application 50 may be configured to accept client connections. It can receive incoming client connection requests and manage the traffic of these requests to the database server.

In one non-limiting example, a listener application 50 may be configured with one or more listening protocol addresses, information about supported services, and parameters that control its runtime behavior. The listener configuration may be stored in a configuration file or the like. In one embodiment, configuration parameters in the listener may have default values. Because all of the configuration parameters have default values, it is possible to start and use a listener with no configuration. This default listener has a name of LISTENER, supports no services upon startup, and listens on a pre-selected TCP/IP protocol address such as but not limited to (ADDRESS=(PROTOCOL=tcp)(HOST=host_name)(PORT=800)). In at least one embodiment, the listener application 50 may be run on a platform and/or device that is separate, physically and/or functionally, from a database in the LIS 30.

Referring still to Figure 9, one non-limiting example of a listener application 50 architecture is described herein. In this non-limiting example, the database server may be configured to receive an initial connection from a client application through the listener application 50. In one embodiment, the listener application 50 may be an application positioned on top of the foundation layer. Figure 9 illustrates the various layers on the client and database server during an initial connection. Although the listener application 50 in this non-limiting example shows that it is part of the database server, it should also be understood that in other system configurations, the listener application 50 may be running on a hardware platform separate (physically and/or functionally) from the database server.

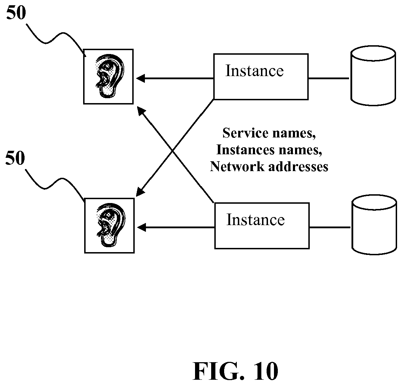

Referring now to Figure 10, this embodiment of the listener application 50 may be configured to broker client requests, handing off the requests to the database server. Every time a client requests a network session with a database server, a listener application 50 may receive the initial request. Each listener application 50 may be configured with one or more protocol addresses that specify its listening endpoints. Optionally, clients configured with one of these protocol addresses can send connection requests to the listener application 50.

Once a client request has reached the listener application 50, the listener application 50 may select an appropriate service handler to service the client's request and forwards the client's request to it. Optionally, the listener application 50 may determine if a database service and its service handlers are available through service registration. During service registration, the process monitor (PMON) process--an instance background process-may provide the listener application 50 with information such as but not limited to the following: a) names of the database services provided by the database; b) name of the instance associated with the services and its current and maximum load; and/or c) service handlers (dispatchers and dedicated servers) available for the instance, including their type, protocol addresses, and current and maximum load.

In this non-limiting example, this information can enable the listener application 50 to direct a client's request appropriately as seen Figure 10, which shows instances registering information with listener application(s) 50. Note that Figure 10 does not represent all the information that can be registered. It should also be understood that some embodiments of the system can be configured to have more than one listener application 50 associated with an LIS 30. Some embodiments, for example, may have two listener applications 50 that can both communicate with the database of the LIS 30. Optionally, some embodiments, for example, may have three or more listener applications 50 associated with the LIS 30.

Optionally, listening endpoints--port numbers--may be dynamically registered with the listener application 50. For example, with XML DB, HTTP, FTP, and WebDAV listening endpoints may be registered with the listener application 50.

If the listener application 50 is not running when an instance starts, PMON may not be able to register the service information. Optionally, PMON attempts to connect periodically to the listener application 50. However, in some scenarios, it may take a period of startup time before PMON registers with the listener application 50 after it has been started. To initiate service registration immediately after the listener application 50 is started, one may use the SQL statement ALTER SYSTEM REGISTER. This is especially useful in high-availability configurations. If a listener application 50 receives an incoming request before the respective instance has been registered, the listener may reject the request.

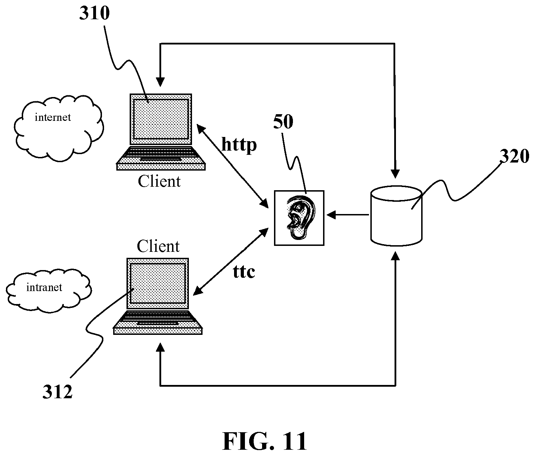

Referring now to Figure 11, this non-limiting example shows one role of a listener application 50 during connection establishment with a browser on client 310 making an HTTP connection (over TCP/IP) and a client 312 making a TTC connection (over TCP/IP) may include: a) the database 320 registers information about the services, instances, and service handlers with the listener application 50; b) the client makes an initial connection with the listener application 50; and/or c) the listener application 50 parses the client request and forwards it to the service handler for the database service requested.

### Database Server Process Architecture

Based on the service handler type registered with the listener application 50, the listener application 50 may forward requests to either a shared server or dedicated server process.

### Shared Server Processes

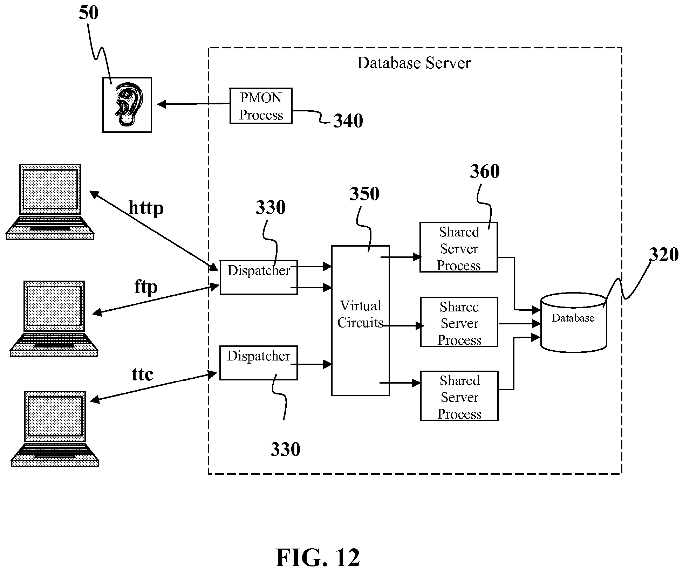

In one non-limiting example, shared server processes may be utilized in the shared server architecture. Figure 12 depicts one embodiment of a shared server architecture. With shared server architectures, client processes may ultimately connect to a dispatcher 330. The PMON 340 process registers the location and load of the dispatchers 330 with the listener application 50, enabling the listener application 50 to forward requests to the least loaded dispatcher 330.

In this non-limiting example, a dispatcher 300 can support multiple client connections concurrently. Each client connection may be bound to a virtual circuit 350. In one embodiment, a virtual circuit 350 may be a piece of shared memory used by the dispatcher 330 for client database connection requests and replies. The dispatcher 330 may place a virtual circuit on a common queue when a request arrives. An idle shared server 360 may pick up the virtual circuit from the common queue, services the request, and relinquishes the virtual circuit before attempting to retrieve another virtual circuit from the common queue. This approach enables a small pool of server processes to serve a large number of clients.

### Dedicated Server Processes

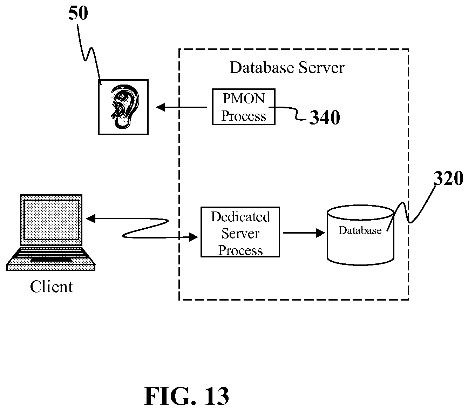

Figure 13 depicts a dedicated server architecture. With a dedicated server architecture, each client process connects to a dedicated server process. In this non-limiting example, the server process is not shared by any other client.

In one non-limiting example, PMON 340 registers information about dedicated server processes with the listener application 50. This enables the listener application 50 to start up a dedicated server process when a client request arrives and forward the request to it.

Optionally, a database listener application 50 may be a database process which "listen" for users (clients) connecting to the database. The listener process, either creates a dedicated server process for each user or to a shared multithreaded process that handles many users.

### Remote listener versus local listener

Optionally, the system further comprises a listener application 50 which optionally: monitors all or substantially incoming messages to the business servlet and the plurality of servlets or the LIS 30; checks where each incoming message is bound; and if the listener application 50 does not recognize the destination, the listener application 50 can allow the message to pass to a default destination.

The World Wide Web includes a network of servers on the Internet, each of which is associated with one or more HTML (Hypertext Markup Language) pages. The HTML pages associated with a server provide information and hypertext links to other documents on that and (usually) other server. Servers communicate with clients by using the Hypertext Transfer Protocol (HTTP). The servers listen for requests from clients for their HTML pages, and are therefore often referred to as "listeners".

Users of the World Wide Web use a client program, referred to as a browser, to request, decode and display information from listeners. When the user of a browser selects a link on an HTML page, the browser that is displaying the page sends a request over the Internet to the listener associated with the Universal Resource Locator (URL) specified in the link. In response to the request, the listener transmits the requested information to the browser that issued the request. The browser receives the information, presents the received information to the user, and awaits the next user request.

### Authentication

Authentication may include the process of identifying the end-users in a transaction as well as the series of steps to be executed before identity can be confirmed. Authentication can be utilized whenever a secure transaction is initiated between a principle and a recipient, such as a client request to access a secure site.

In one embodiment, it may be desirable that the perception to the LIS is that to the that all the devices are "local" in the sense that they provide data to the LIS as if they were part of the local system physically coupled by wired connections to the LIS but are instead coupled to the LIS through a data network comprising components such as but not limited to a LAN, WAN, or external computer processor(s) that may define a "cloud" network. Authentication can be used to assist in implementation of such a distributed network of SPU devices. A listener application which could run (with or without UI) on a hardware platform such as but not limited to a router, a computer, a tablet, or other computer processor that may be developed in the future, can be used to route authenticated data to the LIS from associated and/or authenticated SPUs. In one nonlimiting example, the listener 50 may comprise at least one router such as that available from Cisco, Inc. which is modified to include a further software application, instance, or the like to include the listener function along with the data connectivity capability of the router.

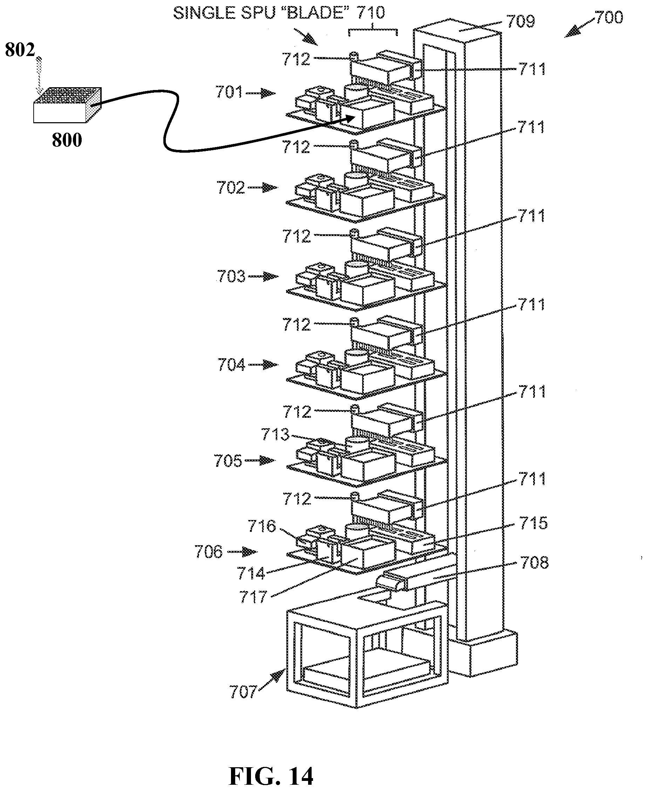

One exemplary embodiment of a workflow for implementing such an LIS and distributed network of SPUs comprises verification of at least one or more of the following: authenticity of the data, authenticity of the device itself so that the device sending the data is authentic, technician authentication, location authentication, and/or other forms of authentication to provide a level of trust that the SPU is authenticated. The verification may be performed all at one location or can be handled by multiple components of the network. In one non-limiting example, it is desirable to only have verified data released from the cloud 110. Some embodiments may use a combination of certifications, keys, encryption, and/or secure data connections such as but not limited to VPNs to verify data. Some embodiments may also verify that any controls and/or calibrators run with that cartridge (as described in Figure 14) can also be checked to see if they are in the expected range of values.

For increase security purposes, in some embodiments, there is no patient data stored on the sample processing units. There may be bar code data associated with samples, but in at least one embodiment, no patient data is on the sample, in the sample identifier, or on the SPU. Optionally, some embodiments may include such information if authorized by the laboratory.

In one non-limiting example, the laboratory will have control before test results are released and displayed. Some may allow the results to be displayed at the sample processing device. Some embodiments may optionally allow for patient data to be displayed at the sample processing device (in addition to sample results). In at least one embodiment, this may be remotely controlled by the LIS and/or cloud 110 as to what information can be displayed and where. In one embodiment, data travels to the LIS and the results can be displayed in a manner and/or location dictated by the user. In one example, it will only be released from LIS after verification by an authorizer such as a laboratory director or other authorized authority. Some may set this to be an automated process handled by a computer processor if certain criteria are met to release test results or other data.

Some embodiments may have the user select which tests are to be run at the SPU and then the system communicates to the user which cartridge 800 to select to insert into the SPU (see Figure 14). Optionally, some embodiments may reverse the workflow and have the user insert a cartridge at which point the system communicates to the user which tests can be run by that cartridge 800. Some may have the option to run all tests that can be done on the cartridge.

In one non-limiting example, configuration of the SPU in relation to an LIS could occur through the LIS, through cloud 110, or at directly at the SPU. For example, a first SPU could be configured be assigned by the cloud or LIS so that there is an association between the SPU and the LIS system. This could be useful if the LIS system is already registered or otherwise included in the database. This may facilitate setup in terms of information and secure connection, as an LIS registered with the cloud will already have established a track record of with certain already-authenticated component(s). In one non-limiting example, the location of the device can be placed in ER, hospital room, intensive care unit, or other location. For the second SPU in this example, the SPU may be sent to the destination without pre-configuration at the cloud or LIS. Once connected to a network, at least one or more authentication steps such by entering user ID and password, can be used so that the cloud and/or listener can recognize the machine. It should be understood that other authentication known or to be developed can also be used to establish a confirmed identity. By way of non-limiting example, some may have user ID badges, biometrics, genetic ID, etc....to ensure that a user is authorized. Once authenticated, the SPU can be associated with a particular LIS. They have virtually added the device to their LIS. For the third SPU of this example, the SPU can be instructed to be part of a particular LIS by manual setup at the SPU. A user ID and password or other credentials can be used to authenticate the credentials of the party trying to create this configuration for the SPU. If the user is verified, then the device can be configured as manually instructed by the user to be associated with an SPU. There may still be limitations in that manual instructions to associate an SPU beyond the LIS or other boundaries associated with the user will typically not be complete. Once configured with an LIS, this SPU is no longer location dependent and can be used anywhere in the world while still sending data to be associated with a particular LIS.

In one non-limiting example, the cloud can sort incoming data and have it routed to the associated LIS. A traditional LIS configuration does not have authentication as presently described and simply trusts that the analyzer, because of the short physical connection and location in a laboratory is sufficient. The traditional analyzers faithfully trust that the data is being handed to the correct and authorized receiver, without further verification.

For at least some of the embodiments herein, data coming to the cloud is encrypted and typically contains a detailed data header, metadata, or other information for proper processing. In one non-limiting example, such information may include information about the SPU sending the data, the destination, the manufacturer of the SPU, calibration information, control information, associated LIS information, healthcare network information, HMO information, or other information to assist in linking the data with the desired destination. Incremental decryption may be used to verify data being sent by an SPU to the cloud 110 and/or to a listener 50. One embodiment may initially determine if the data is coming from an authentic source. Another embodiment may then verify that the authentic source is a particular type of device such as but not limited to a particular manufacturer or other device characteristic. Optionally, only a certain brand of device can encrypt the data that the cloud system with a corresponding key or other factor can decrypt such as but not limited to public private key. Optionally, various factors can be reviewed to determine if there was any tampering that was detected. Then the data is reviewed to see if the device was authorized to run this particular cartridge, wherein at least some cartridges may have a bar code or the like. Optionally, the system can verify if the test was authorized to be run on behalf of (or by) a particular laboratory. Optionally, the system can verify if the device was at a location that is part of the particular laboratory or healthcare system (or at another authorized location). Optionally, the system can verify if the user that was authenticated was one who has privileges such as being part of an authorized user group that is allowed to operate the device. Optionally, some of the embodiments may only run at least two of foregoing authorization. Optionally, some of the embodiments may only run at least three of foregoing authorization. Optionally, some of the embodiments may allow an administrator to select and/or deselect the verification steps for the data. All of the foregoing may occur using one or more components that may be part of the cloud 110.

Optionally, the interpretation and/or verification of the data can be based on issues with controls and/or calibrators associated with a particular cartridge 800 or reagent. For example, some may have each cartridge with its own barcode or other identifier (unique, class, or otherwise). Based on the results from controls and/or calibrators associated with the cartridge, then the results of such controls and/or calibrators run for that cartridge can be verified for expected results. If the system was tampered with, the controls and/or calibrators will show that something was not correctly processed. If they are off, then the data will be rejected. Most traditional systems only run controls and/or calibrators once in the morning, not with every cartridge or at some selected time interval or number of cartridges.

Optionally, with the integrity of the system verified and the user authenticated, the data can be processed. The results may be pushed, pulled, or otherwise made available to the listener. In one embodiment, the listener is configured to be connected by at least one type of secure connection such as but not limited to virtual private network (VPN). Other secure connections to be developed in the future are not excluded. There may already have been a secure handshake and the system may be sometimes connected or always connected to the cloud.

In at least one embodiment, the listener 50 may receive encrypted data wherein only the listener has the key that can decrypt the sent data. The system can also weigh other factors such as but not limited whether the system was expecting this data, did it come from a reliable source, or other checks to determine if it will accept the data. The system can be setup as a push, pull, or full duplex push-pull. Some embodiments may have the configuration where it only accepts data when it requests it, such as but not limited to checking for new data at specific time intervals.

Again for increased security, patient information is residing in the LIS and processing of sample at the SPU and/or by cloud 110 does not require patient information. In one embodiment, it is barcodes for cartridge ID and sample ID that is used to handle information until data reaches the LIS. Additionally, even this is also encrypted in at least one or more embodiments described herein.

This configuration reduces the number of physical connections to an LIS due to the universal listener 50 which may be in a secure physical area where authentication and certification is additionally used on the data stream to the listener so that one can confirm that the information can be trusted.

### Certificate Authentication

Certificate authentication may include the process of identifying the end-users in a transaction as well as the series of steps to be executed before identity can be confirmed. The certificate authentication process identifies users by virtue of their issued certificates, and is utilized whenever a secure transaction is initiated between a principle and a recipient, such as a client request to access a secure site.

Upon initial request, the domain's server may present its digital certificate to the client with its public key and verified credentials. Certificate authentication is not concerned so much with these items as it is the signature of the issuing certificate authority. This signature is what the client browser will validate against its cache of recognized and trusted certificates and library of certificate authorities. If accepted, then certificate authentication is successful. If the issuing certificate authority is not recognized, then the certificate is not authenticated and instead, the user receives notification that the credentials supplied were invalid.

When client browsers verify digital certificates, they are checking to see that the certificate has been signed by a trusted certificate authority. This signature is the most important component of a certificate. Before a certificate will be issued and signed by a certificate authority, the domain must be registered and the owner's credentialing information must be verified. Once endorsed, however, the certificate becomes a unique and unchangeable document that is suitable only for its holder. As seen in Figures 4 to 5B, certificate authentication can be used along one or more portions of the data pathways described in one or more embodiments herein.

### Sample Processing Unit

Figure 14 shows one exemplary embodiment of a system **700** having a plurality of modules **701-706** and a cytometry station **707,** in accordance with an embodiment of the invention. The plurality of modules include a first module **701,** second module **702,** third module **703,** fourth module **704,** fifth module **705** and sixth module **706.**

Figure 14 shows a cartridge 800 with at least one information storage unit such as a barcode, QR code, RFID, NFC, or other information storage unit therewith. It should be understood that information storage units that may be developed in the future can also be adapted for use with the cartridge 800. In one nonlimiting example, the cartridge may contain all reagent units and all assay units use for analyzing a biological sample for the presence of at least two analytes. In one non-limiting example, at least one calibrator is included in the cartridge.. In one non-limiting example, at least one control is included in the cartridge. In one non-limiting example, at least one calibrator and at least one control are included in the cartridge. In one non-limiting example, at least two calibrators and at least one control are included in the cartridge. In one non-limiting example, at least one calibrator and at least two controls are included in the cartridge. The cartridge 800 may include fluid transfer disposables, reagent vessels, mixing vessels, diluent vessels, and/or the like that may be used by a sample handling system 708. It may optionally include all pipette tips, reagents, and movable assay units used for processing at least two assays, optionally with at least one diluent and/or one included separately on the SPU. This non-limiting example may allow for almost all reagents and disposables to be part of the cartridge except for perhaps one, two, or single digit number of commonly used ones which can be stored on the SPU. Optionally, all reagents and disposables are part of the cartridge and are removed with the cartridge when it is ejected from the SPU. The cartridge may form a used disposables container (due to having a lid in many embodiments) for controlled and contained disposal of used components, including those that may include residual sample.

Figure 14 shows that at least one container 802 for at least one biological sample may be included as part of cartridge 800 that is inserted into the sample processing unit SPU as indicated by the arrow. Optionally, some embodiments may insert one or more containers 802 directly into the SPU.

In one non-limiting example, the cytometry station **707** is operatively coupled to each of the plurality of modules **701-706** by way of a sample handling system **708.** The sample handling system **708** may include a pipette, such as a positive displacement, air displacement or suction-type pipette, as described herein. Other details about system 700 can be found in U.S. Patent application Ser. No. 13/769,820 filed Feb. 18, 2013.

The cytometry station **707** includes a cytometer for performing cytometry on a sample, as described above and in other embodiments of the invention. The cytometry station **707** may perform cytometry on a sample while one or more of the modules **701-706** perform other preparation and/or assaying procedure on another sample. In some situations, the cytometry station **707** performs cytometry on a sample after the sample has undergone sample preparation in one or more of the modules **701-706.**

The system **700** includes a support structure **709** having a plurality of bays (or mounting stations). The plurality of bays is for docking the modules **701-706** to the support structure **709.** The support structure **709,** as illustrated, is a rack.

Each module is secured to rack **709** with the aid of an attachment member. In an embodiment, an attachment member is a hook fastened to either the module or the bay. In such a case, the hook is configured to slide into a receptacle of either the module or the bay. In another embodiment, an attachment member includes a fastener, such as a screw fastener. In another embodiment, an attachment member is formed of a magnetic material. In such a case, the module and bay may include magnetic materials of opposite polarities so as to provide an attractive force to secure the module to the bay. In another embodiment, the attachment member includes one or more tracks or rails in the bay. In such a case, a module includes one or more structures for mating with the one or more tracks or rails, thereby securing the module to the rack **709.** Optionally, power may be provided by the rails.

An example of a structure that may permit a module to mate with a rack may include one or more pins. In some cases, modules receive power directly from the rack. In some cases, a module may be a power source like a lithion ion, or fuel cell powered battery that powers the device internally. In an example, the modules are configured to mate with the rack with the aid of rails, and power for the modules comes directly from the rails. In another example, the modules mate with the rack with the aid of attachment members (rails, pins, hooks, fasteners), but power is provided to the modules wirelessly, such as inductively (i.e., inductive coupling).

In some embodiments, a module mating with a rack need not require pins. For example, an inductive electrical communication may be provided between the module and rack or other support. In some instances, wireless communications may be used, such as with the aid of ZigBee communications or other communication protocols.

Each module may be removable from the rack **709.** In some situations, one module is replaceable with a like, similar or different module. In an embodiment, a module is removed from the rack **709** by sliding the module out of the rack. In another embodiment, a module is removed from the rack **709** by twisting or turning the module such that an attachment member of the module disengages from the rack **709.** Removing a module from the rack **709** may terminate any electrical connectivity between the module and the rack **709.**

In an embodiment, a module is attached to the rack by sliding the module into the bay. In another embodiment, a module is attached to the rack by twisting or turning the module such that an attachment member of the module engages the rack **709.** Attaching a module to the rack **709** may establish an electrical connection between the module and the rack. The electrical connection may be for providing power to the module or to the rack or to the device from the module and/or providing a communications bus between the module and one or more other modules or a controller of the system **700.**

Each bay of the rack may be occupied or unoccupied. As illustrated, all bays of the rack **709** are occupied with a module. In some situations, however, one or more of the bays of the rack **709** are not occupied by a module. In an example, the first module **701** has been removed from the rack. The system **700** in such a case may operate without the removed module.

In some situations, a bay may be configured to accept a subset of the types of modules the system **700** is configured to use. For example, a bay may be configured to accept a module capable of running an agglutination assay but not a cytometry assay. In such a case, the module may be "specialized" for agglutination. Agglutination may be measured in a variety of ways. Measuring the time-dependent change in turbidity of the sample is one method. One can achieve this by illuminating the sample with light and measuring the reflected light at 90 degrees with an optical sensor, such as a photodiode or camera. Over time, the measured light would increase as more light is scattered by the sample. Measuring the time dependent change in transmittance is another example. In the latter case, this can be achieved by illuminating the sample in a vessel and measuring the light that passes through the sample with an optical sensor, such as a photodiode or a camera. Over time, as the sample agglutinates, the measured light may reduce or increase (depending, for example, on whether the agglutinated material remains in suspension or settles out of suspension). In other situations, a bay may be configured to accept all types of modules that the system **700** is configured to use, ranging from detection stations to the supporting electrical systems.

Each of the modules may be configured to function (or perform) independently from the other modules. In an example, the first module **701** is configured to perform independently from the second **702,** third **703,** fourth **704,** fifth **705** and sixth **706** modules. In other situations, a module is configured to perform with one or more other modules. In such a case, the modules may enable parallel processing of one or more samples. In an example, while the first module **701** prepares a sample, the second module **702** assays the same or different sample. This may enable a minimization or elimination of downtime among the modules.

The support structure (or rack) **709** may have a server type configuration. In some situations, various dimensions of the rack are standardized. In an example, spacing between the modules **701-706** is standardized as multiples of at least about 0.5 inches, or 1 inch, or 2 inches, or 3 inches, or 4 inches, or 5 inches, or 6 inches, or 7 inches, or 8 inches, or 9 inches, or 10 inches, or 11 inches, or 12 inches.

The rack **709** may support the weight of one or more of the modules **701-706.** Additionally, the rack **709** has a center of gravity that is selected such that the module **701** (top) is mounted on the rack **709** without generating a moment arm that may cause the rack **709** to spin or fall over. In some situations, the center of gravity of the rack **709** is disposed between the vertical midpoint of the rack and a base of the rack, the vertical midpoint being 50% from the base of the rack **709** and a top of the rack. In an embodiment, the center of gravity of the rack **709,** as measured along a vertical axis away from the base of the rack **709,** is disposed at least about 0.1%, or 1%, or 10%, or 20%, or 30%, or 40%, or 50%, or 60%, or 70%, or 80%, or 90%, or 100% of the height of the rack as measured from the base of the rack **709.**

A rack may have multiple bays (or mounting stations) configured to accept one or more modules. In an example, the rack **709** has six mounting stations for permitting each of the modules **701-706** to mount the rack. In some situations, the bays are on the same side of the rack. In other situations, the bays are on alternating sides of the rack.

In some embodiments, the system **700** includes an electrical connectivity component for electrically connecting the modules **701-706** to one another. The electrical connectivity component may be a bus, such as a system bus. In some situations, the electrical connectivity component also enables the modules **701-706** to communicate with each other and/or a controller of the system **700.**

In some embodiments, the system **700** includes a controller (not shown) for facilitating processing of samples with the aid of one or more of the modules **701-706.** In an embodiment, the controller facilitates parallel processing of the samples in the modules **701-706.** In an example, the controller directs the sample handling system **708** to provide a sample in the first module **701** and second module **702** to run different assays on the sample at the same time. In another example, the controller directs the sample handling system **708** to provide a sample in one of the modules **701-706** and also provide the sample (such as a portion of a finite volume of the sample) to the cytometry station **707** so that cytometry and one or more other sample preparation procedures and/or assays are done on the sample in parallel. In such fashion, the system minimizes, if not eliminates, downtime among the modules **701-706** and the cytometry station **707.**

Each individual module of the plurality of modules may include a sample handling system for providing samples to and removing samples from various processing and assaying modules of the individual module. In addition, each module may include various sample processing and/or assaying modules, in addition to other components for facilitating processing and/or assaying of a sample with the aid of the module. The sample handling system of each module may be separate from the sample handling system **708** of the system **700.** That is, the sample handling system **708** transfers samples to and from the modules **701-706,** whereas the sample handling system of each module transfers samples to and from various sample processing and/or assaying modules included within each module.

In the illustrated example of Figure 14, the sixth module **706** includes a sample handling system **710** including a suction-type pipette **711** and positive displacement pipette **712.** The sixth module **706** includes a centrifuge **713,** a spectrophotometer **714,** a nucleic acid assay (such as a polymerase chain reaction (PCR) assay) station **715** and PMT **716.** An example of the spectrophotometer **714** is shown in Figure 140 (see below). The sixth module **706** further includes a cartridge **717** for holding a plurality of tips for facilitating sample transfer to and from each processing or assaying module of the sixth module.

In an embodiment, the suction type pipette **711** includes 1 or more, or 2 or more, or 3 or more, or 4 or more, or 5 or more, or 6 or more, or 7 or more, or 8 or more, or 9 or more, or 10 or more, or 15 or more, or 20 or more, or 30 or more, or 40 or more, or 50 or more heads. In an example, the suction type pipette **711** is an 8-head pipette with eight heads. The suction type pipette **711** may be as described in other embodiments of the invention.

In some embodiments, the positive displacement pipette **712** has a coefficient of variation less than or equal to about 20%, 15%, 12%, 10%, 9%, 8%, 7%, 6%, 5%, 4%, 3%, 2%, 1%, 0.5%, 0.3%, or 0.1% or less. The coefficient of variation is determined according to σ/µ, wherein 'σ' is the standard deviation and 'µ' is the mean across sample measurements.

In an embodiment, all modules are identical to one another. In another embodiment, at least some of the modules are different from one another. In an example, the first, second, third, fourth, fifth, and sixth modules **701-706** include a positive displacement pipette and suction-type pipette and various assays, such as a nucleic acid assay and spectrophotometer. In another example, at least one of the modules **701-706** may have assays and/or sample preparation stations that are different from the other modules. In an example, the first module **701** includes an agglutination assay but not a nucleic acid amplification assay, and the second module **702** includes a nucleic acid assay but not an agglutination assay. Modules may not include any assays.

In the illustrated example of Figure 14, the modules **701-706** include the same assays and sample preparation (or manipulation) stations. However, in other embodiments, each module includes any number and combination of assays and processing stations described herein.

The modules may be stacked vertically or horizontally with respect to one another. Two modules are oriented vertically in relation to one another if they are oriented along a plane that is parallel, substantially parallel, or nearly parallel to the gravitational acceleration vector. Two modules are oriented horizontally in relation to one another if they are oriented along a plane orthogonal, substantially orthogonal, or nearly orthogonal to the gravitational acceleration vector.

In an embodiment, the modules are stacked vertically, i.e., one module on top of another module. In the illustrated example of Figure 14, the rack **709** is oriented such that the modules **701-706** are disposed vertically in relation to one another. However, in other situations the modules are disposed horizontally in relation to one another. In such a case, the rack **709** may be oriented such that the modules **701-706** may be situated horizontally alongside one another.

It should be understood that, like the embodiment of Figure 14, modules 701-704 may all be modules that are identical to one another. In another embodiment, at least some of the modules are different from one another. In an example, the first, second, third, and/or fourth modules 701-704 may be replaced by one or more other modules that can occupy the location of the module being replaced. The other modules may optionally provide different functionality such as but not limited to a replacing one of the modules 701-704 with one or more cytometry modules 707, communications modules, storage modules, sample preparation modules, slide preparation modules, tissue preparation modules, or the like. For example, one of the modules 701-704 may be replaced with one or more modules that provide a different hardware configuration such as but not limited to provide a thermal controlled storage chamber for incubation, storage between testing, and/or storage after testing. Optionally, the module replacing one or more of the modules 701-704 can provide a non-assay related functionality, such as but not limited to additional telecommunication equipment for the system 730, additional imaging or user interface equipment, or additional power source such as but not limited to batteries, fuel cells, or the like. Optionally, the module replacing one or more of the modules 701-704 may provide storage for additional disposables and/or reagents or fluids. It should also be understood that configurations may also be run with not every bay or slot occupied by a module, particularly in any scenario wherein one or more types of modules draw more power that other modules. In such a configuration, power otherwise directed to an empty bay can be used by the module that may draw more power than the others.

In one non-limiting example, each module is secured to the support structure 732 with the aid of an attachment member. In an embodiment, an attachment member is a hook fastened to either the module or the bay. In such a case, the hook is configured to slide into a receptacle of either the module or the bay. In another embodiment, an attachment member includes a fastener, such as a screw fastener. In another embodiment, an attachment member is formed of a magnetic material. In such a case, the module and bay may include magnetic materials of opposite polarities so as to provide an attractive force to secure the module to the bay. In another embodiment, the attachment member includes one or more tracks or rails in the bay. In such a case, a module includes one or more structures for mating with the one or more tracks or rails, thereby securing the module to the support structure 732. Optionally, power may be provided by the rails.

An example of a structure that may permit a module to mate with a support structure 732 may include one or more pins. In some cases, modules receive power directly from the support structure 732. In some cases, a module may be a power source like a lithium ion, or fuel cell powered battery that powers the device internally. In an example, the modules are configured to mate with the support structure 732 with the aid of rails, and power for the modules comes directly from the rails. In another example, the modules mate with the support structure 732 with the aid of attachment members (rails, pins, hooks, fasteners), but power is provided to the modules wirelessly, such as inductively (i.e., inductive coupling).

In some embodiments, the modules **701-706** are in communication with one another and/or a controller of the system **700** by way of a communications bus ("bus"), which may include electronic circuitry and components for facilitating communication among the modules and/or the controller. The communications bus includes a subsystem that transfers data between the modules and/or controller of the system **700.** A bus may bring various components of the system **700** in communication with a central processing unit (CPU), memory (e.g., internal memory, system cache) and storage location (e.g., hard disk) of the system **700.**

A communications bus may include parallel electrical wires with multiple connections, or any physical arrangement that provides logical functionality as a parallel electrical bus. A communications bus may include both parallel and bit-serial connections, and can be wired in either a multidrop (i.e., electrical parallel) or daisy chain topology, or connected by switched hubs. In an embodiment, a communications bus may be a first generation bus, second generation bus or third generation bus. The communications bus permits communication between each of the modules and other modules and/or the controller. In some situations, the communications bus enables communication among a plurality of systems, such as a plurality of systems similar or identical to the system **700.**

The system **700** may include one or more of a serial bus, parallel bus, or self-repairable bus. A bus may include a master scheduler that control data traffic, such as traffic to and from modules (e.g., modules **701-706**), controller, and/or other systems. A bus may include an external bus, which connects external devices and systems to a main system board (e.g., motherboard), and an internal bus, which connects internal components of a system to the system board. An internal bus connects internal components to one or more central processing units (CPUs) and internal memory.

In some embodiments, the communication bus may be a wireless bus. The commuincations bus may be a Firewire (IEEE 1394), USB (1.0, 2.0, 3.0, or others), Lightning, or Thunderbolt.

In some embodiments, the system **700** includes one or more buses selected from the group consisting of Media Bus, Computer Automated Measurement and Control (CAMAC) bus, industry standard architecture (ISA) bus, USB bus, Firewire, Thunderbolt, extended ISA (EISA) bus, low pin count bus, MBus, MicroChannel bus, Multibus, NuBus or IEEE 1196, OPTi local bus, peripheral component interconnect (PCI) bus, Parallel Advanced Technology Attachment (ATA) bus, Q-Bus, S-100 bus (or IEEE 696), SBus (or IEEE 1496), SS-50 bus, STEbus, STD bus (for STD-80 [8-bit] and STD32 [16-/32-bit]), Unibus, VESA local bus, VMEbus, PC/104 bus, PC/104 Plus bus, PC/104 Express bus, PCI-104 bus, PCIe-104 bus, 1-Wire bus, HyperTransport bus, Inter-Integrated Circuit (I²C) bus, PCI Express (or PCIe) bus, Serial ATA (SATA) bus, Serial Peripheral Interface bus, UNI/O bus, SMBus, 2-wire or 3-wire interface, self-repairable elastic interface buses and variants and/or combinations thereof.

In some situations, the system **700** includes a Serial Peripheral Interface (SPI), which is an interface between one or more microprocessors and peripheral elements or I/O components (e.g., modules **701-706**) of the system **700.** The SPI can be used to attach 2 or more, or 3 or more, or 4 or more, or 5 or more, or 6 or more, or 7 or more, or 8 or more, or 9 or more, or 10 or more or 50 or more or 100 or more SPI compatible I/O components to a microprocessor or a plurality of microprocessors. In other instances, the system **700** includes RS-485 or other standards.

In an embodiment, an SPI is provided having an SPI bridge having a parallel and/or series topology. Such a bridge allows selection of one of many SPI components on an SPI I/O bus without the proliferation of chip selects. This is accomplished by the application of appropriate control signals, described below, to allow daisy chaining the device or chip selects for the devices on the SPI bus. It does however retain parallel data paths so that there is no Daisy Chaining of data to be transferred between SPI components and a microprocessor.

In some embodiments, an SPI bridge component is provided between a microprocessor and a plurality of SPI I/O components which are connected in a parallel and/or series (or serial) topology. The SPI bridge component enables parallel SPI using MISO and MOSI lines and serial (daisy chain) local chip select connection to other slaves (CSL/). In an embodiment, SPI bridge components provided herein resolve any issues associated with multiple chip selects for multiple slaves. In another embodiment, SPI bridge components provided herein support four, eight, sixteen, thirty two, sixty four or more individual chip selects for four SPI enabled devices (CS1/ - CS4/). In another embodiment, SPI bridge components provided herein enable four times cascading with external address line setting (ADR0 - ADR1). In some situations, SPI bridge components provided herein provide the ability to control up to eight, sixteen, thirty two, sixty four or more general output bits for control or data. SPI bridge components provided herein in some cases enable the control of up to eight, sixteen, thirty two, sixty four or more general input bits for control or data, and may be used for device identification to the master and/or diagnostics communication to the master.

### Device calibration and/or maintenance

In some embodiments the device may be capable of performing on-board calibration and/or controls. The device may be capable of performing one or more diagnostic step (e.g., preparation step and/or assay step). If the results fall outside an expected range, a portion of the device may be cleaned and/or replaced. The results may also be useful for calibrating the device. On-board calibration and/or controls may occur without requiring human intervention. Calibration and controls may occur within a device housing.

A device may also be capable of performing on-board maintenance. If during a calibration, operation of device, diagnostic testing, or any other point in time a condition requiring repair and/or maintenance of the device is detected, the device may institute one or more automated procedures to perform said maintenance and/or repair. Any description of maintenance may include repair, cleaning, and/or adjustments. For example, a device may detect that a component is loose and may automatically tighten the component. The device may also detect that a wash or diluents level is running low in a module and provide an alert to add more wash or diluents, or bring over wash or diluents from another module.

The system may be configured to continue to function after the removal and/or failure of certain modules.

Calibration and/or maintenance may occur on a periodic basis. In some embodiments, device calibration and/or maintenance may automatically occur at regular or irregular intervals. Device calibration and/or maintenance may occur when one or more condition is detected from the device. For example, if a component appears to be faulty, the device may run a diagnostic on associated components. Device calibration and/or maintenance may occur at the instruction of an operator of the device. Device calibration and/or maintenance may also occur upon automated instruction from an external device. The calibration and quality control (QC) cartridge is briefly described in the next paragraph. The goal of the calibration cartridge is to enable the quantitative assessment and adjustment of each module/detector of the device. For example, by performing a variety of assay steps, functionality is tested/evaluated for the pipette, gantry, centrifuge, cameras, spectrometer, nucleic acid amplification module, thermal control unit, and cytometer. Each measurement made during calibration cartridge runs with reagent controls may be compared to device requirements for precision. By way of non-limiting example, there is a pass fail outcome for these results. If re-calibration is required, the data generated is used to recalibrate the device (such as the device sensors and pipettes). Recalibration ensures that each device is accurate. Some QC can also be performed automatically in the device without introducing a cartridge. For example, the light sources in the device can be used to periodically QC the optical sensors in the device. An external device or control may maintain a device calibration schedule and/or device maintenance schedule for a plurality of devices. Device calibration and/or maintenance may occur on a time-based schedule or a use-based schedule. For example, devices that are used more frequently than others may be calibrated and/or maintained more frequently and/or vice versa. QC data may be indexed with data stored, for example, on the sample processing device or an external device.

In some embodiments, a calibration protocol may be stored on a sample processing device, or on an external device and transmitted from the external device to the sample processing device. In some embodiments, a sample processing device may communicate with an external device to provide QC data to the external device. In some embodiments, the external device may send a protocol or calibration instructions to a sample processing device based on QC data provided from the sample processing device to the external device.

In some embodiments, the device may be periodically calibrated and quality controlled. Each module, consisting of one or more hardware units, could be calibrated periodically by utilizing a calibration cartridge. The calibration cartridge may consist of a series of standard fluids, which a properly calibrated system gives a known response to. The module results to these standards could be read, analyzed and based on deviations or absence thereof, module status can be determined, and corrected for, if necessary. The calibration standards could either be stored in the device or introduced separately as a cartridge.

In some embodiments, some modules may auto-correct for any changes in the environment. For example, temperature sensors on the pipette may automatically trigger an adjustment in the required piston movement, to correct for temperature fluctuations. In general, modules where feedback regarding performance is available, may auto-correct for any changes over time.

In some embodiments, the output measurements of the cytometer may be calibrated to match results from predicate devices or devices utilizing other technologies as required.

In embodiments, a device may monitor its environment, including its internal and external environment. In embodiments, a device may provide device environmental information to a laboratory. Device environmental information includes, e.g., internal temperature, external temperature, internal humidity, external humidity, time, status of components, error codes, images from an internal camera, images from an external camera, and other information. In some embodiments, a device may contain a thermal sensor. In embodiments, an internal camera may be fixed at an internal location. In embodiments, an internal camera may be fixed at an internal location and may be configured to rotate, scan, or otherwise provide views of multiple areas or regions within the device. In embodiments, an internal camera may be movable within the device; for example, an internal camera may be mounted on a movable element, such as a pipette, within the device. In embodiments, an internal camera may be movable within the device and may be configured to rotate, scan, or otherwise provide multiple views of areas within the device from multiple locations within the device. In embodiments, an external camera may be fixed at an external location. In embodiments, an external camera may be fixed at an external location and may be configured to rotate, scan, or otherwise provide multiple views of areas outside the device. In embodiments, an external camera may be movable on or around the outside of the device. In embodiments, an external camera may be movable and may be configured to rotate, scan, or otherwise provide multiple views of areas outside the device from multiple locations on or around the outside of the device.

Transmission of device environmental information to a laboratory is useful for the oversight and control of the device, including being useful for the oversight and control of the dynamic operation of the device. Transmission of device environmental information to a laboratory is useful for maintaining the integrity of the operation and control of the device, quality control of the operation and control of the device, and for reducing variation or error in the data collection and sample processing performed by the device. For example, transmission of temperature information to a laboratory is useful for the oversight and control of the device, and is useful in the analysis by the laboratory of data provided by the device to the laboratory. For example, a device may have dedicated temperature zones, and this information may be transmitted to a laboratory.

In embodiments, a device may be configured to control the temperature within the device, or within a portion of the device. The device or portion thereof may be maintained at a single constant temperature, or at a progression of different selected temperatures. Such control improves the reproducibility of measurements made within the device, may unify or provide regularity of conditions for all samples, and reduce the variability of measurements and data, e.g., as measured by the coefficient of variance of multiple measurements or replicate measurements. Such control may als affect chemistry performance in the assay(s) and speed/kinetics of the assay reaction. Temperature information may be useful for quality control. In embodiments, a device may monitor temperature and control its internal temperature. Temperature control may be useful for quality control. A device that monitors and controls its temperature may transmit temperature information to a laboratory; a laboratory may use such temperature information in the control of the operation of the instrument, in the oversight of the instrument, and in the analysis of data transmitted from the instrument. Temperature control may also be used for regulating the speed of assays performed with the device. For example, a device may be maintained at a temperature which optimizes the speed of one or more selected assays (e.g. at 20° C, 22° C, 25° C, 27° C, 32° C, 35° C, 37 ° C, 40 ° C, 42 ° C, 45° C, 47° C, 50 ° C,52° C, 55° C, 57° C, 60° C, 62° C, 65° C, 67° C, 70 ° C, 72° C, 75° C, 77° C, 80 ° C, 82° C, 85° C, 87° C, 90 ° C, 92° C, 95° C, or 97° C).

In embodiments, a device may be configured to acquire images from within the device, or within a portion of the device. Such images may provide information about the position, condition, availability, or other information regarding components, reagents, supplies, or samples within the device, and may provide information used in control of the operation of the device. Such images may be useful for quality control. A device that acquires images from within the device may transmit image information to a laboratory; a laboratory may use such image information in the control of the operation of the instrument, possibly dynamically or in real-time continuously or in real-time but in select intervals, in the oversight of the instrument, and in the analysis of data transmitted from the instrument.

### Device Security

One or more security features may be provided on a sample processing device. The device may have one or more motion sensor that may determine when the device changes orientation or is moved. The device may be able to detect if someone is trying to open the device. For example one or more sensor may detect if portions of the device are taken apart. The device may be able to detect if the device falls or is tipped over. The device may be able to sense any motion of the device or any motion near the device. For example, the device may be able to sense if an object or person gets within a certain distance of the device (e.g., using motion sensors, optical sensors, thermal sensors, and/or audio sensors). The device may be able to determine if the device is unplugged or if an error occurs on the device. Any description of actions that may occur as a result of device tampering may be applied to any other device condition as described herein, and vice versa. Accelerometer(s), vibration sensor(s), and/or tilt sensor(s) are used to determine rapid movements and jarring of the device. Optionally, cameras on the outside of the device can image and recognize their surroundings and/or provide security to the device in terms of video capture, sounding an alert, or only providing access to verified individual(s) or device(s).

In some embodiments, an alert may be provided if someone is trying to open a device, or if someone comes within the device's proximity. In some instances, an alert may be provided if the device housing is breached. Similarly, an alert may be provided if the device falls, tips over, or if an error is detected. The device may encompass a stabilization system with, optionally, shock absorbance and dampening capabilities to prevent it from tipping when for example moving in vehicles at high speeds. In some instances, if the device detects that the device is being opened, approached, or tampered with, a camera on the device may capture an image of the device surroundings. The device may capture an image of the individual trying to open the device. The data associated with the device may be sent to the cloud or an external device. The device associated with the tampering of the device, such as an image of an individual tampering with the device may be transmitted from the device. The data associated with the device, which may include one or more image, may be stored in the device. In the event that the device is not able to immediately transmit the data, the data may be transmitted once the device is able and/or connected to a network.

The device may include one or more microphone or audio detection device that may be able to record and/or relay sound. For example if a device is tampered with, the microphone may collect audio information and the audio information may be stored on the device or may be transmitted from the device.

Optionally, the device may include one or more location sensing device. For example, the device may have a GPS tracker within the device. When any tampering with the device is detected, the location of the device may be transmitted from the device. The location may be transmitted to an external device or the cloud. In some instances, the location of the device may be continuously broadcast once the tampering is detected, or may be transmitted at one or more intervals or other detected events. An owner or entity associated with the device may be able to track the location of the device. In some instances, a plurality of location sensors may be provided so that even the device is taken apart and/or one or more location sensor is found and destroyed, it may be possible to track other parts of the device. In the event that the device is unable to transmit the device location at a particular moment, the device may be able to store the device location and transmit it once it is able.

In some embodiments, the device may be designed so that it can only be opened from the inside, or be designed to be only opened from the inside. For example, in some embodiments the device does not have fasteners or screws on the outside of the device. Any mechanical fastening and/or opening features may be on the inside of the device. The device may be mechanically locked from inside the housing. The external portion of the housing may include no exterior fastening/locking mechanisms. The device may be opened from the inside upon one or more instructions from a controller. For example, the device may have one or more touchscreen or other user interface that may accept an instruction from a user for the device to open. The device may have one or more communication unit that may receive an instruction from an external device for the device to open. Based on said instructions, one or more opening mechanism within the device may cause the device to open. In some instances, the device may require electrical power for the device to open. In some instances, the device may only when plugged in. Alternatively, the device may open when powered by a local energy storage system or energy generation system. In some instances, the device may only open if it receives instructions from a user who has been identified and/or authenticated. For instance, only certain users may be granted the authority to cause the device to open.

The device may have one or more local energy storage system. The energy storage system may permit one or more portions of the device to operate even if the device is separated from an external energy source. For example, if the device is unplugged, one or more energy storage system may permit one or more portion of the device to operate. In some instances, the energy storage system may permit all parts of the device to operate. In other examples, the local energy storage system may permit certain information to be transmitted from the device to the cloud. The local energy storage may be sufficient to power a camera that may capture one or more image of the device surroundings and/or an individual tampering with the device. The local energy storage may be sufficient to power a GPS or other location sensor that may indicate the location of the device. The local energy storage may be sufficient to save and/or transmit the state of the device e.g., in a log-based journaling approach so that the device can pick up where it left off or know what steps need to be performed. The local energy storage may be sufficient to power a transmission unit that may send information relating to the device to the cloud and/or an external device.

In one embodiment, the device and the external controller maintain a security mechanism by which no unauthorized person with physical access to the device may be able to retrieve test information and link it back to an individual, thus protecting the privacy of patient health data. An example of this would be where the device captures user identification information, send it to the external device or cloud, receives a secret key from the cloud and erases all patient information from the device. In such a scenario, if the devices send any further data about that patient to the external device, it will be referred to link through the secret key already obtained from the external device.

### Computer Architecture

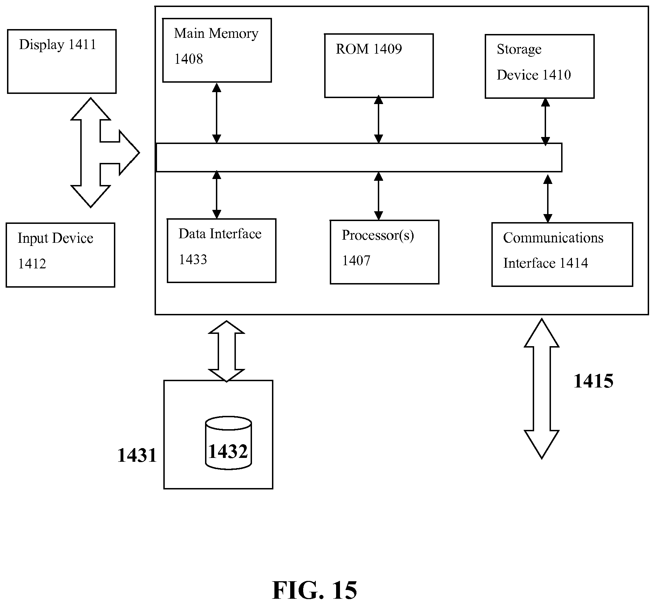

The execution of the database sequences of instructions to practice the embodiments herein such as server 120 and/or at the listener 50 may be performed by a computer system 1400 as shown in FIG. 15. In one non-limiting embodiment, execution of the sequences of instructions is performed by a single computer system 1400. According to other embodiments, two or more computer systems 1400 coupled by a communication link 1415 may perform the sequence of instructions in coordination with one another. Although a description of only one computer system 1400 will be presented below, however, it should be understood that any number of computer systems 1400 may be employed to practice the embodiments.

A computer system 1400 according to an embodiment will now be described with reference to FIG. 15, which is a block diagram of the functional components of a computer system 1400. As used herein, the term computer system 1400 is broadly used to describe any computing device that can store and independently run one or more programs.

Each computer system 1400 may include a communication interface 1414 coupled to the bus 1406. The communication interface 1414 provides two-way communication between computer systems 1400. The communication interface 1414 of a respective computer system 1400 transmits and receives electrical, electromagnetic or optical signals, that include data streams representing various types of signal information, e.g., instructions, messages and data. A communication link 1415 links one computer system 1400 with another computer system 1400. For example, the communication link 1415 may be a LAN, in which case the communication interface 1414 may be a LAN card, or the communication link 1415 may be a PSTN, in which case the communication interface 1414 may be an integrated services digital network (ISDN) card or a modem, or the communication link 1415 may be the Internet, in which case the communication interface 1414 may be a dial-up, cable or wireless modem.

A computer system 1400 may transmit and receive messages, data, and instructions, including program, i.e., application, code, through its respective communication link 1415 and communication interface 1414. Received program code may be executed by the respective processor(s) 1407 as it is received, and/or stored in the storage device 1410, or other associated non-volatile media, for later execution.

In an embodiment, the computer system 1400 operates in conjunction with a data storage system 1431, e.g., a data storage system 1431 that contains a database 1432 that is readily accessible by the computer system 1400. The computer system 1400 communicates with the data storage system 1431 through a data interface 1433. A data interface 1433, which is coupled to the bus 1406, transmits and receives electrical, electromagnetic or optical signals, that include data streams representing various types of signal information, e.g., instructions, messages and data. In embodiments, the functions of the data interface 1433 may be performed by the communication interface 1414.

Computer system 1400 includes a bus 1406 or other communication mechanism for communicating instructions, messages and data, collectively, information, and one or more processors 1407 coupled with the bus 1406 for processing information. Computer system 1400 also includes a main memory 1408, such as a random access memory (RAM) or other dynamic storage device, coupled to the bus 1406 for storing dynamic data and instructions to be executed by the processor(s) 1407. The main memory 1408 also may be used for storing temporary data, i.e., variables, or other intermediate information during execution of instructions by the processor(s) 1407.

The computer system 1400 may further include a read only memory (ROM) 1409 or other static storage device coupled to the bus 1406 for storing static data and instructions for the processor(s) 1407. A storage device 1410, such as a magnetic disk or optical disk, may also be provided and coupled to the bus 1406 for storing data and instructions for the processor(s) 1407.

A computer system 1400 may be coupled via the bus 1406 to a display device 1411, such as, but not limited to, a cathode ray tube (CRT), for displaying information to a user. An input device 1412, e.g., alphanumeric and other keys, is coupled to the bus 1406 for communicating information and command selections to the processor(s) 1407.

According to one embodiment, an individual computer system 1400 performs specific operations by their respective processor(s) 1407 executing one or more sequences of one or more instructions contained in the main memory 1408. Such instructions may be read into the main memory 1408 from another computer-usable medium, such as the ROM 1409 or the storage device 1410. Execution of the sequences of instructions contained in the main memory 1408 causes the processor(s) 1407 to perform the processes described herein. In alternative embodiments, hard-wired circuitry may be used in place of or in combination with software instructions. Thus, embodiments are not limited to any specific combination of hardware circuitry and/or software.

The term "computer-usable medium," as used herein, refers to any medium that provides information or is usable by the processor(s) 1407. Such a medium may take many forms, including, but not limited to, non-volatile and volatile media. Non-volatile media, i.e., media that can retain information in the absence of power, includes the ROM 1409, CD ROM, flash memory, solid state memory, magnetic tape, and magnetic discs. Volatile media, i.e., media that cannot retain information in the absence of power, includes the main memory 1408. Logic refers to software, hardware or any combination of software and hardware.

While the invention has been described and illustrated with reference to certain particular embodiments thereof, those skilled in the art will appreciate that various adaptations, changes, modifications, substitutions, deletions, or additions of procedures and protocols may be made without departing from the spirit and scope of the invention. For example, with any of the above embodiments, it should be understood that some embodiments may consider the LIS 30 and the listener application 50 to be part of the same LIS system, wherein the listener and/or broker application can be viewed in one embodiment as a gateway to the LIS system. Although systems herein are described in the context of LIS, it should also be understood that embodiments herein can also be configured for use with systems such as but not limited to Laboratory Information Management System (LIMS), Laboratory Management System (LMS), or Process Development Execution System (PDES).

Additionally, concentrations, amounts, and other numerical data may be presented herein in a range format. It is to be understood that such range format is used merely for convenience and brevity and should be interpreted flexibly to include not only the numerical values explicitly recited as the limits of the range, but also to include all the individual numerical values or sub-ranges encompassed within that range as if each numerical value and sub-range is explicitly recited. For example, a size range of about 1 nm to about 200 nm should be interpreted to include not only the explicitly recited limits of about 1 nm and about 200 nm, but also to include individual sizes such as 2 nm, 3 nm, 4 nm, and sub-ranges such as 10 nm to 50 nm, 20 nm to 100 nm, etc....

The publications discussed or cited herein are provided solely for their disclosure prior to the filing date of the present application. Nothing herein is to be construed as an admission that the present invention is not entitled to antedate such publication by virtue of prior invention. Further, the dates of publication provided may be different from the actual publication dates which may need to be independently confirmed. The following application is referred to : U.S. Provisional Application Ser. No. 61/858,604 filed July 25 2013.

In this document, the terms "computer program medium" and "computer usable medium" are used to generally refer to media such as, for example, memory, storage unit, media, and channel. These and other various forms of computer program media or computer usable media may be involved in carrying one or more sequences of one or more instructions to a processing device for execution. Such instructions embodied on the medium, are generally referred to as "computer program code" or a "computer program product" (which may be grouped in the form of computer programs or other groupings). When executed, such instructions might enable the computing module to perform features or functions of the present invention as discussed herein.

While various embodiments of the present invention have been described above, it should be understood that they have been presented by way of example only, and not of limitation. Likewise, the various diagrams may depict an exemplary architectural or other configuration for the invention, which is done to aid in understanding the features and functionality that can be included in the invention. The embodiments herein are not restricted to the illustrated exemplary architectures or configurations, but the desired features can be implemented using a variety of alternative architectures and configurations. Indeed, it will be apparent to one of ordinary skill in the art how alternative functional, logical or physical partitioning and configurations can be applied to implement the desired features of the present invention. Also, a multitude of different constituent module names other than those depicted herein can be applied to the various partitions. Additionally, with regard to flow diagrams, operational descriptions and method claims, the order in which the steps are presented herein shall not mandate that various embodiments be implemented to perform the recited functionality in the same order unless the context dictates otherwise. Additionally, although the listener 50 is shown as separate from the LIS, it should be understood that some embodiments may configure hardware to run both LIS and listener on the same computer, device, or hardware platform. Although the embodiments herein are described in the context of an LIS, it should be understood that the listener and/or other features may be adapted for use with other healthcare or non-healthcare related data systems currently known or to be developed in the future.

Terms and phrases used in this document, and variations thereof, unless otherwise expressly stated, should be construed as open ended as opposed to limiting. As examples of the foregoing: the term "including" should be read as meaning "including, without limitation" or the like: the term "example" is used to provide exemplary instances of the item in discussion, not an exhaustive or limiting list thereof; the terms "a" or "an" should be read as meaning "at least one," "one or more" or the like; and adjectives such as "conventional," "traditional," "normal," "standard," "known" and terms of similar meaning should not be construed as limiting the item described to a given time period or to an item available as of a given time, but instead should be read to encompass conventional, traditional, normal, or standard technologies that may be available or known now or at any time in the future. Likewise, where this document refers to technologies that would be apparent or known to one of ordinary skill in the art, such technologies encompass those apparent or known to the skilled artisan now or at any time in the future.

The presence of broadening words and phrases such as "one or more," "at least," "but not limited to" or other like phrases in some instances shall not be read to mean that the narrower case is intended or required in instances where such broadening phrases may be absent. The use of the term "module" does not imply that the components or functionality described or claimed as part of the module are all configured in a common package. Indeed, any or all of the various components of a module, whether control logic or other components, can be combined in a single package or separately maintained and can further be distributed in multiple groupings or packages or across multiple locations.

Additionally, the various embodiments set forth herein are described in terms of exemplary block diagrams, flow charts and other illustrations. As will become apparent to one of ordinary skill in the art after reading this document, the illustrated embodiments and their various alternatives can be implemented without confinement to the illustrated examples. For example, block diagrams and their accompanying description should not be construed as mandating a particular architecture or configuration.

This document contains material subject to copyright protection. The copyright owner (Applicant herein) has no objection to facsimile reproduction of the patent documents and disclosures, as they appear in the US Patent and Trademark Office patent file or records, but otherwise reserves all copyright rights whatsoever. The following notice shall apply: Copyright 2013-2014 Theranos, Inc.

While preferred embodiments of the present invention have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein may be employed in practicing the invention. Any feature, whether preferred or not, may be combined with any other feature, whether preferred or not. The appended claims are not to be interpreted as including means-plus-function limitations, unless such a limitation is explicitly recited in a given claim using the phrase "means for." It should be understood that as used in the description herein and throughout the claims that follow, the meaning of "a," "an," and "the" includes plural reference unless the context clearly dictates otherwise. For example, a reference to "an assay" may refer to a single assay or multiple assays. Also, as used in the description herein and throughout the claims that follow, the meaning of "in" includes "in" and "on" unless the context clearly dictates otherwise. Finally, as used in the description herein and throughout the claims that follow, the meaning of "or" includes both the conjunctive and disjunctive unless the context expressly dictates otherwise. Thus, the term "or" includes "and/or" unless the context expressly dictates otherwise.

## Claims

1. A method for use with a laboratory information system, LIS, comprising:
receiving sample data at the LIS from a database, wherein the sample data originates from at least one sample processing unit, SPU, at a physical location remote from a physical location of the LIS and wherein the database is resident on a computing device at a location remote from the LIS,
wherein the sample data traverses at least one data pathway through one or more wide area networks before reaching a data network comprising the LIS;
**characterized in that** the method comprises:
receiving comprises retrieving data from an intermediate device having a database, wherein the intermediate device receives the sample data from said at least one sample processing unit;
wherein said at least one sample processing unit is associated on-demand with a health plan laboratory.

2. The method of claim 1 further comprising:
authenticating sample data from the sample processing unit for at least two factors before processing the sample data for the database.

3. The method of claim 1 further comprising:
processing the sample data to provide processed sample data that is stored in the database, wherein processed sample data is sent to the LIS.

4. The method of claim 1 further comprising:
processing the sample data to provide interpretations of the sample data for storage in the database.

5. The method of claim 1 further comprising:
using data from at least one assay calibrator to be one factor in authenticating sample data authenticity.

6. The method of claim 1 further comprising:
using data from at least one control to be one factor in authenticating sample data authenticity, wherein the control comprises a component known to provide a predetermined result.

7. The method of claim 1 further comprising:
using a listener application operably in communication with the LIS and the database to receive the sample data from the database and then transfer to the LIS.

8. The method according to claim 1 wherein the sample data is received at a listener application operably in communication with the LIS, wherein the sample data originates from at least one sample processing unit at a physical location remote from a physical location of the LIS and wherein sample data is sent to a database resident on a computing device at a location remote from the LIS, and the sample data received by the listener application is sent from the database.

9. The method according to claim 1 further comprising:
monitoring at least one indicator that sample data for a biological sample associated with a laboratory has been uploaded to a database from at least one of a plurality of sample processing units (SPUs).

10. The method according to claim 1 for use with a clinical laboratory data management system comprising:
monitoring at least one database that sample data associated with the clinical laboratory has been uploaded to the database, wherein said sample data originates from at least one of a plurality of sample processing units (SPUs) at a physical location remote from a physical location of the clinical laboratory data management system;
receiving said sample data from the database in an electronic format at the clinical laboratory data management system, wherein the database is on a computing device at a location remote from the physical location of the clinical laboratory data management system,
wherein the sample data is sent along a data pathway through one or more wide area networks before reaching a data network comprising the clinical laboratory data management system;
wherein receiving comprises retrieving data from an intermediate device having a database, wherein the intermediate device receives the sample data from said at least one sample processing unit;
wherein said at least one sample processing unit is associated on-demand with a health plan laboratory.

11. The method according to claim 1 or 10 further comprising:
receiving sample data in an electronic format at a laboratory information system (LIS), wherein the data originates from at least one sample processing unit at a location remote from a physical location of the laboratory information system and wherein the data traverses one or more wide area networks before reaching the laboratory information system;
verifying integrity of the sample data;
ensuring that the sample data is in a laboratory information system file format at a point in time after the sample data has been received by the laboratory information system; and
storing data into the electronic medical records system by way of an interface between an electronic medical record system and the laboratory information system, wherein storing the data further comprises ensuring that the data is in an electronic medical record system (EMR) file format that is different from the LIS file format.

12. The method of claim 4 wherein: the sample processing units are physically distant from one another, and wherein a data pathway to the laboratory information system from at least one of the sample processing units comprises traversing: a) at least one wide area network, b) a central database collecting said sample processing data, and c) a listener application configured to process sample processing data from the central database for paired sample processing unit data.

13. The method of claim 4 wherein: verifying comprises using public key private encryption and decryption.

14. The method of claim 4 wherein:
verifying comprises using at least one certificate for certificate authentication.

## Patentansprüche

1. Ein Verfahren für die Nutzung mit einem Laborinformationssystem, LIS, umfassend:
das Empfangen von Probendaten im LIS aus einer Datenbank, wobei die Probendaten von mindestens einer Probenverarbeitungseinheit, SPU, an einem physischen Standort stammen, der von einem physischen Standort des LIS entfernt ist, und wobei die Datenbank auf einem Computergerät an einem Standort untergebracht ist, der vom LIS entfernt ist,
wobei die Probendaten mindestens einen Datenpfad durch ein oder mehrere Weitverkehrsnetze durchlaufen bevor sie ein Datennetz erreichen, das das LIS beinhaltet;
**gekennzeichnet dadurch, dass** das Verfahren Folgendes umfasst:
Das Empfangen umfasst das Abrufen der Daten von einem Zwischengerät mit einer Datenbank, wobei das Zwischengerät die Probendaten von der besagten mindestens einen Probenverarbeitungseinheit empfängt;
wobei die mindestens eine Probenverarbeitungseinheit On-Demand mit einem Gesundheitsplan-Labor verbunden ist.

2. Verfahren nach Anspruch 1, ferner umfassend:
Das Authentifizieren der Probendaten aus der Probenverarbeitungseinheit für mindestens zwei Faktoren bevor die Probendaten für die Datenbank verarbeitet werden.

3. Verfahren nach Anspruch 1, ferner umfassend:
Das Verarbeiten der Probendaten, um verarbeitete Probendaten bereitzustellen, die in der Datenbank gespeichert sind, wobei die verarbeiteten Probendaten an das LIS geschickt werden.

4. Verfahren nach Anspruch 1, ferner umfassend:
Das Verarbeiten der Probendaten, um Interpretationen der Probendaten für die Speicherung in der Datenbank bereitzustellen.

5. Verfahren nach Anspruch 1, ferner umfassend:
Das Nutzen der Daten aus mindestens einem Assay-Kalibrator, als ein Faktor bei der Authentifizierung der Probendaten-Authentizität.

6. Verfahren nach Anspruch 1, ferner umfassend:
Das Nutzen der Daten aus mindestens einer Steuerung, als ein Faktor bei der Authentifizierung der Probendaten-Authentizität, wobei die Steuerung eine Komponente beinhaltet, die bekannt ist, ein vorbestimmtes Resultat bereitzustellen.

7. Verfahren nach Anspruch 1, ferner umfassend:
Das Nutzen einer Listener-Anwendung, die betriebsbereit ist, mit dem LIS und der Datenbank zu kommunizieren, um die Probendaten von der Datenbank zu empfangen und dann an das LIS zu übertragen.

8. Verfahren nach Anspruch 1, wobei
die Probendaten in einer Listener-Anwendung empfangen werden, die in Verbindung mit dem LIS betriebsbereit ist, wobei die Probendaten von mindestens einer Probenverarbeitungseinheit an einem physischen Standort stammen, der von einem physischen Standort des LIS entfernt ist, und wobei die Probendaten an eine Datenbank geschickt werden, die auf einem Computergerät an einem Standort untergebracht ist, das vom LIS entfernt ist, und die Probendaten, die von der Listener-Anwendung empfangen werden, werden von der Datenbank geschickt.

9. Verfahren nach Anspruch 1, ferner umfassend:
Das Überwachen mindestens eines Indikators, dass Probendaten für eine biologische Probe, die mit einem Labor verbunden ist, in eine Datenbank von mindestens einer aus einer Vielzahl von Probenverarbeitungseinheiten (SPUs) hochgeladen wurde.

10. Das Verfahren nach Anspruch 1 für die Nutzung in einem klinischen Labor-Datenmanagementsystem, umfassend:
Das Überwachen mindestens einer Datenbank, dass Probendaten, die mit dem klinischen Labor verbunden sind, in die Datenbank hochgeladen wurden, wobei die besagten Probendaten von mindestens einer aus einer Vielzahl von Probenverarbeitungseinheiten (SPUs) an einem physischen Standort stammen, der von einem physischen Standort des klinischen Labor-Datenmanagementsystems entfernt ist;
das Empfangen der besagten Probendaten von der Datenbank in einem elektronischen Format im klinischen Labor-Datenmanagementsystem, wobei sich die Datenbank auf einem Computergerät an einem Standort befindet, der von dem physischen Standort des klinischen Labor-Datenmanagementsystems entfernt ist,
wobei die Probendaten entlang eines Datenpfads durch ein oder mehrere Weitverkehrsnetze geschickt werden bevor sie ein Datennetz erreichen, das das klinische Labor-Datenmanagementsystem beinhaltet;
wobei das Empfangen das Abrufen der Daten von einem Zwischengerät mit einer Datenbank umfasst, wobei das Zwischengerät die Probendaten von der besagten mindestens einen Probenverarbeitungseinheit empfängt;
wobei die mindestens eine Probenverarbeitungseinheit On-Demand mit einem Gesundheitsplan-Labor verbunden ist.

11. Das Verfahren nach Anspruch 1 oder 10, ferner umfassend:
Das Empfangen der Probendaten in einem elektronischen Format in einem Laborinformationssystem (LIS), wobei die Daten von mindestens einer Probenverarbeitungseinheit an einem Standort stammen, der von einem physischen Standort des Laborinformationssystems entfernt ist, und wobei die Daten ein oder mehrere Weitverkehrsnetze durchlaufen bevor sie das Laborinformationssystem erreichen;
das Verifizieren der Vollständigkeit der Probendaten;
das Sicherstellen, dass die Probendaten in einem Dateiformat des Laborinformationssystems zu einem Zeitpunkt vorliegen, der nach dem Empfang der Probendaten durch das Laborinformationssystem liegt; und
das Speichern der Daten in dem elektronischen Krankenaktensystem über eine Schnittstelle zwischen einem elektronischen Krankenaktensystem und dem Laborinformationssystem, wobei das Speichern der Daten ferner beinhaltet, dass die Daten in einem Dateiformat des elektronischen Krankenaktensystems (EMR) vorliegen, das sich von dem Dateiformat des LIS unterscheidet.

12. Das Verfahren nach Anspruch 4, wobei:
Die Probenverarbeitungseinheiten physisch voneinander entfernt sind, und wobei ein Datenpfad zum Laborinformationssystem von mindestens einer der Probenverarbeitungseinheiten das Durchlaufen folgender Pfade umfasst: a) mindestens ein Weitverkehrsnetz, b) eine zentrale Datenbank, die diese Probenverarbeitungsdaten erfasst, und c) eine Listener-Anwendung, die konfiguriert ist, die Probenverarbeitungsdaten von der zentralen Datenbank für gepaarte Daten der Probenverarbeitungseinheit zu verarbeiten.

13. Das Verfahren nach Anspruch 4, wobei:
Das Verifizieren die Nutzung von Public Key-Private Key-Verschlüsselung und -Entschlüsselung umfasst.

14. Das Verfahren nach Anspruch 4, wobei:
Das Verifizieren die Nutzung mindestens eines Zertifikats für die Zertifikat-Authentifizierung umfasst.

## Revendications

1. Procédé destiné à une utilisation avec un système d'information de laboratoire, LIS, comprenant:
la réception de données d'échantillon sur le LIS en provenance d'une base de données, dans lequel les données d'échantillon proviennent d'au moins une unité de traitement d'échantillon, SPU, à un emplacement physique distant d'un emplacement physique du LIS et dans lequel la base de données est résidente sur un dispositif informatique dans un lieu distant du LIS,
dans lequel les données d'échantillon traversent au moins un chemin de données par l'intermédiaire d'un ou plusieurs réseaux étendus avant de parvenir à un réseau de données comprenant le LIS ;
**caractérisé en ce que** le procédé comprend :
la réception comprend l'extraction des données à partir d'un dispositif intermédiaire possédant une base de données, dans lequel le dispositif intermédiaire reçoit les données d'échantillon provenant d'au moins une unité de traitement d'échantillon ;
dans lequel ladite moins une unité de traitement d'échantillon est associée à un laboratoire de plan de santé à la demande.

2. Procédé selon la revendication 1 comprenant :
l'authentification des données d'échantillon provenant de l'unité de traitement d'échantillon sur au moins deux facteurs avant le traitement des données d'échantillon destinées à la base de données.

3. Procédé selon la revendication 1 comprenant :
le traitement des données d'échantillon afin de fournir des données d'échantillon traitées qui sont mémorisées dans la base de données, dans lequel les données d'échantillon traitées sont envoyées au LIS.

4. Procédé selon la revendication 1 comprenant :
le traitement des données d'échantillon afin de fournir les interprétations des données d'échantillon destinées à la mémorisation dans la base de données.

5. Procédé selon la revendication 1 comprenant :
à l'aide de données provenant d'au moins un étalonneur de dosage, devant être un facteur dans l'authentification de l'authenticité des données d'échantillon.

6. Procédé selon la revendication 1 comprenant :
à l'aide de données provenant d'au moins un contrôle devant être un facteur dans l'authentification de l'authenticité des données d'échantillon, dans lequel le contrôle comprend un élément connu pour fournir un résultat prédéfini.

7. Procédé selon la revendication 1 comprenant :
à l'aide d'une application d'écoute utilisable en communication avec le LIS et la base de données pour recevoir les données d'échantillon provenant de la base de données et ensuite les transférer au LIS.

8. Procédé selon la revendication 1 dans lequel
les données d'échantillon sont reçues dans une application d'écoute utilisable en communication avec le LIS, dans lequel les données d'échantillon proviennent d'au moins une unité de traitement d'échantillon à un emplacement physique distant d'un emplacement physique du LIS et dans lequel les données d'échantillon sont envoyées à une base de données résidente sur un dispositif informatique dans un emplacement distant du LIS et les données d'échantillon reçues par l'application d'écoute sont envoyées à partir de la base de données.

9. Procédé selon la revendication 1 comprenant en outre :
la surveillance d'au moins un indicateur que les données d'échantillon destinées à un échantillon biologique associé à un laboratoire ont été téléchargées vers l'amont dans une base de données à partir d'au moins une pluralité d'unités de traitement d'échantillon (SPU).

10. Procédé selon la revendication 1 destiné à une utilisation pour une utilisation avec un système de gestion des données de laboratoire clinique comprenant :
la surveillance d'au moins une base de données que les données d'échantillon associées au laboratoire clinique ont été téléchargées ers l'amont dans la base de données, dans lequel lesdites données d'échantillon proviennent d'au moins un échantillon d'une pluralité d'unités de traitement d'échantillon (SPU) à un emplacement physique distant d'un emplacement physique du système de gestion des données de laboratoire clinique ;
la réception des dites données d'échantillon provenant de la base de données dans un format électronique sur le système de gestion des données de laboratoire clinique, dans lequel la base de données est sur un dispositif informatique à un emplacement distant de l'emplacement physique du système de gestion des données de laboratoire clinique,
dans lequel les données d'échantillon sont envoyées le long d'un chemin de données par l'intermédiaire d'un ou plusieurs réseaux étendus avant de parvenir à un réseau de données comprenant le système de gestion des données de laboratoire clinique ;
dans lequel la réception comprend l'extraction des données à partir d'un dispositif intermédiaire possédant une base de données, dans lequel le dispositif intermédiaire reçoit les données d'échantillon provenant d'au moins une unité de traitement d'échantillon ;
dans lequel ladite moins une unité de traitement d'échantillon est associée à un laboratoire de plan de santé à la demande.

11. Procédé selon la revendication 1 ou 10 comprenant en outre :
la réception des données d'échantillon dans un format électronique sur un système d'information de laboratoire (LIS), dans lequel les données proviennent d'au moins une unité de traitement d'échantillon à un emplacement distant de l'emplacement physique d'un système d'information de laboratoire et dans lequel les données traversent un ou plusieurs réseaux étendus avant d'atteindre le système d'information de laboratoire ;
la vérification de l'intégrité des données d'échantillon ;
l'assurance que les données d'échantillon sont dans un format de fichier du système d'information de laboratoire à un point dans le temps après que les données d'échantillon ont été reçues par le système d'information de laboratoires ; et
la mémorisation des données dans le système de dossiers médicaux électroniques au moyen d'une interface entre un système de dossiers médicaux électroniques et le système d'information de laboratoire, dans lequel la mémorisation des données comprend en outre l'assurance que les données sont dans un format de fichier du système de dossiers médicaux électroniques (EMR) qui est différent du format de fichier du LIS.

12. Procédé selon la revendication 4, dans lequel :
les unités de traitement d'échantillon sont physiquement à distance les unes des autres et dans lequel un chemin de données vers le système d'information de laboratoire d'au moins une des unités de traitement d'échantillon comprend la traversée : a) d'au moins un réseau étendu, b) d'une base de données centrale collectant lesdites données de traitement d'échantillon et c) d'une application d'écoute configurée pour traiter les données de traitement d'échantillon provenant de la base de données centrale pour les données de l'unité de traitement d'échantillons appariés.

13. Procédé selon la revendication 4, dans lequel :
la vérification comprend l'utilisation de chiffrement et de déchiffrement à clés publique ot privée.

14. Procédé selon la revendication 4, dans lequel :
la vérification comprend l'utilisation d'au moins un certificat pour l'authentification par certificat.
